# EUROPÄISCHE PATENTSCHRIFT

(11) **EP 2 599 433 B1**
(45) Veröffentlichungstag und Bekanntmachung des Hinweises auf die Patenterteilung: **11.03.2020**
(21) Anmeldenummer: 12007918.1
(22) Anmeldetag: 23.11.2012
(51) Int. Cl.: A61B 1/05, A61B 5/107, G01S 17/10, G01S 17/89, G02B 23/24, A61B 1/00, A61B 5/00, A61B 5/06, A61B 34/20, A61B 1/04, A61B 90/00

(54) **VORRICHTUNG ZUR ENDOSKOPISCHEN 3D-DATENERFASSUNG**
DEVICE FOR ENDOSCOPIC 3D DATA ACQUISITION
DISPOSITIF D'ACQUISITION DE DONNÉES 3D ENDOSCOPIQUE

(30) Priorität: 29.11.2011 DE 102011119608
(43) Veröffentlichungstag der Anmeldung: 05.06.2013
(73) Patentinhaber: Karl Storz SE & Co. KG, 78532 Tuttlingen (DE)
(72) Erfinder: BAUMANN, Harald, 78532 Tuttlingen (DE); IRION, Klaus M., 78532 Tuttlingen (DE)
(74) Vertreter: Weidner Stern Jeschke

(56) Entgegenhaltungen:
- EP-A1- 2 108 306
- EP-A1- 2 260 784
- EP-A1- 2 551 698
- WO-A1-00/49938
- WO-A1-97/29684
- WO-A1-98/29032
- WO-A1-2005/077272
- WO-A1-2007/115825
- DE-A1- 19 800 765
- DE-A1-102006 017 003
- DE-A1-102009 013 761
- US-A1- 2002 165 448
- US-A1- 2008 097 156
- US-A1- 2011 288 373
- -: "Weltkoordinatensystem", WIKIPEDIA, 3 November 2011 (2011-11-03), XP055316090,
- GROCH A ET AL: "3D surface reconstruction for laparoscopic computer-assisted interventions: comparison of state-of-the-art methods", MEDICAL IMAGING 2011: VISUALIZATION, IMAGE-GUIDED PROCEDURES, AND MODELING, SPIE, 1000 20TH ST. BELLINGHAM WA 98225-6705 USA, vol. 7964, no. 1, 1 March 2011 (2011-03-01), pages 1-9, XP060008083, DOI: 10.1117/12.878354 [retrieved on 2011-03-01]

## Beschreibung

Die vorliegende Erfindung betrifft eine Vorrichtung zur endoskopischen 3D-Datenerfassung. Viele medizinische Untersuchungen und chirurgische Operationen werden heute endoskopisch durchgeführt. Hierdurch kann die Belastung des Patienten erheblich verringert werden. Aufgrund des durch den endoskopischen Zugang eingeschränkten Blickfeldes erfordern endoskopische Operationen jedoch ein erhebliches Maß an Übung des Operateurs, um die Entfernung zu einer Oberfläche eines körperinneren Hohlraums, in dem eine chirurgische Manipulation vorgenommen werden soll, richtig einzuschätzen. Aus dem gleichen Grunde ist die Vermessung intrakorporaler Strukturen, etwa die Ermittlung der Größe eines Tumors, mit Schwierigkeiten verbunden. Schließlich kann die durch das eingeschränkte Blickfeld eines Endoskops erschwerte Orientierung des Arztes in dem körperinneren Hohlraum dazu führen, dass bei einer endoskopischen Untersuchung Bereiche der Oberfläche des Hohlraums übersehen werden, d.h. nicht endoskopisch erfasst werden. Für endoskopische Diagnosen und Operationen sind daher die Abstandsermittlung und die Vermessung intrakorporaler Strukturen, ebenso wie die räumliche Erfassung bzw. Rekonstruktion eines intrakorporalen Hohlraums von großer Bedeutung. Hierfür ist die Erfassung von 3D-Daten des Hohlraums erforderlich, insbesondere die Erfassung von absoluten 3D-Daten, die auf ein extrakorporales Referenzkoordinatensystem bezogen sind.

Aus der Offenlegungsschrift DE 10 2006 017 003 A1 ist ein Endoskop zur Tiefenakquisition bekannt, bei dem ein moduliertes Lichtsignal ausgesandt wird und die Modulationsparameter des empfangenen Lichtsignals zur Berechnung der Tiefendaten verwendet werden. Über einen als Strahlteiler verwendeten planen halbdurchlässigen Spiegel können zwei Bildsensoren Strahlung empfangen, wovon einer die zur Erzeugung von 3D-Daten nutzbaren Modulationsparameter aufnimmt, während der andere zur Aufnahme eines visuellen Bildes der endoskopischen Szene vorgesehen ist. In US 2006/0025692 A1 wird eine endoskopische Vorrichtung zur Erzeugung eines endoskopischen Fluoreszenzbildes beschrieben, wobei durch eine Entfernungsmesseinrichtung, die beispielsweise mit Ultraschall, Mikrowellen oder Laserlicht arbeitet, ein Entfernungssignal erzeugt wird. Die Erfassung absoluter 3D-Daten ist bei den genannten Lösungen nicht möglich, so dass die erfassten Daten auf das eingeschränkte Blickfeld des Endoskops begrenzt sind.

In der Offenlegungsschrift DE 10 2008 018 636 A1, die durch Bezugnahme in die vorliegende Anmeldung aufgenommen wird, ist eine Vorrichtung zur endoskopischen 3D-Datenerfassung beschrieben, die Lichterzeugungsmittel zur Erzeugung mindestens einer modulierten Messstrahlung, Lichtweiterleitungsmittel zur Weiterleitung der Messstrahlung auf einen zu beobachtenden Bereich und Lichtabbildungsmittel zur Abbildung einer Signalstrahlung aus dem zu beobachtenden Bereich auf einen phasenempfindlichen Bildsensor umfasst. Durch Auswertung der von dem phasenempfindlichen Bildsensor gelieferten Daten werden 3D-Daten des beobachteten Bereichs erzeugt. Die Erfassung absoluter 3D-Daten ist hierbei nicht vorgesehen.

Aus WO 94/03100 ist ein Verfahren zur Darstellung des Inneren von Körpern bekannt, bei dem ein räumliches Datenfeld zu dem in einer bestimmten Lage befindlichen Körper zugeordnet wird und die räumliche Position einer Kamera, der ein Endoskop vorgeschaltet ist, laufend erfasst wird. Weiterhin werden eine Darstellung des Datenfeldes, die jeweils dem aktuellen Blickwinkel der Kamera entspricht, berechnet und das optische Bild und das Datenfeld gleichzeitig am Monitor dargestellt. Durch einen Eingabevorgang des Anwenders werden ein oder mehrere charakteristische Punkte des Datenfeldes mit der zugehörigen optischen Darstellung am Bildschirm in Übereinstimmung gebracht. Als Datenfeld kann eine dreidimensionale Rekonstruktion verwendet werden, die aus einer oder mehreren zuvor aufgenommenen Videoaufnahmen gewonnen wird, der bzw. denen eine Distanzmessung über Ultraschall bzw. durch stereometrische Analyse zugeordnet ist. Die Ultraschall-Distanzmessung erlaubt jedoch die Erfassung nur relativ weniger Datenpunkte, während eine stereometrische Analyse auf kontrastreiche Oberflächen beschränkt ist. Deshalb und aufgrund der notwendigen Interaktion des Anwenders sind die Anwendbarkeit des Verfahrens und die hierdurch erzielbaren Vorteile eingeschränkt.

In der Patentschrift DE 10 2004 008 164 B3, die durch Bezugnahme in die vorliegende Anmeldung aufgenommen wird, ist eine Vorrichtung zum Erstellen zumindest eines Ausschnitts eines virtuellen 3D-Modells eines Körperinnenraums offenbart, die ein Endoskop, ein Lageerfassungssystem mit einem Trägheitssensorsystem zur Erfassung der Position und Orientierung des Endoskops und ein Abstandsmesssystem zur Erfassung zumindest eines Abstands des Endoskops von zumindest einem Punkt der Oberfläche des Körperinnenraums umfasst. Die Abstandsmessung erfolgt mit Hilfe eines vom Endoskop emittierten Laserstrahls auf der Basis einer Triangulation oder durch Laufzeitmessung des Laserstrahls oder mit Hilfe eines vom Endoskop auf die Oberfläche des Körperinnenraums projizierten Musters oder auch durch Ultraschall. Aus durch das Abstandsmesssystem erfassten Punkten auf der Oberfläche des Körperinnenraums wird ein Ausschnitt eines virtuellen Modells der Oberfläche des Körperinnenraums erstellt. Da hierfür eine Abstandsmessung aus einer Mehrzahl von unterschiedlichen Positionen und Orientierungen des Endoskops notwendig ist, ist nur eine relativ geringe räumliche Auflösung erreichbar.

In dem Artikel von Höller et al., Spatial orientation in translumenal surgery, in: Minimally Invasive Therapy, Vol. 19, 262-273 (2010), wird ein flexibles Endoskop beschrieben, an dessen proximalem Ende ein Time-of-Flight- (TOF-) Sensor angeordnet ist. Am distalen Ende des Endoskops ist ein Trägheitssensor angeordnet, um das endoskopische Bild schwerkraftbasiert aufzurichten bzw. einen korrigierten Bildhorizont zu schaffen. Ein Trägheitssensor benötigt jedoch einen relativ großen Bauraum und ist daher insbesondere bei flexiblen Endoskopen mit geringem Durchmesser nicht ohne Weiteres in einen distalen Endbereich zu integrieren.

In der Offenlegungsschrift DE 10 2009 013 761 A1 ist ein Endoskop mit einem Bildsensor und einem Inertialsensor am distalen Ende des Endoskops beschrieben, wobei der Inertialsensor ausgebildet ist, um eine Verkippung des Bildsensors relativ zum Gravitationsfeld zu erfassen und dadurch unabhängig von einer Orientierung des proximalen Endes des Endoskops eine Bildkorrektur in Bezug auf die Orientierung des distalen Endes zu ermöglichen. Hierfür sind am distalen Ende des Endoskops drei Inertialsensoren angeordnet, die Verkippungssignale erzeugen; aufgrund der daraus berechneten Winkelwerte wird das endoskopische Bild digital gedreht. Eine Position des distalen Endes des Endoskops wird nicht erfasst.

In EP 2 260 784 A1 ist ein System zur Orientierungsunterstützung und Darstellung eines im Körper eines Menschen befindlichen Instruments offenbart, wobei mittels eines Lagemesssystems mehrfache Messungen der 3D-Lage des mit einer oder mehreren Sensoreinheiten versehenen Instruments durchgeführt werden. Aufgrund der 3D-Lagedaten wird ein virtuelles Modell des Instruments generiert und in der Art eines Augmented-Reality-Bildes lage- und größenentsprechend in das aus den Bilddaten generierte Körperoberflächenbild integriert dargestellt. Gemäß WO 98/29032 A1 wird mit Hilfe von Positionssensorelementen, die in einer festen Position zueinander und zu einem distalen Ende eines Katheters an dem Katheter befestigt sind, ein Weg bzw. eine Position des Katheters in einem Hohlraum bestimmt, dessen Form zuvor mit bekannten Verfahren ermittelt worden ist. In US 2002/165448 A1 ist ein Lokalisierungssystem zur Bestimmung des Orts und der Orientierung eines invasiven medizinischen Instruments beschrieben, etwa eines Katheters oder eines Endoskops, wobei in der Nähe des distalen Endes des Instruments eine Mehrzahl von Sensoren angeordnet sind. Eine endoskopische Erfassung dreidimensionaler Daten der Oberfläche eines körperinneren Hohlraums ist nicht vorgesehen.

In der nicht vorveröffentlichten Patentanmeldung EP 2 551 698 A1 ist ein endoskopisches Instrument mit einem optischen Sensor und einem Lagesensor offenbart, welcher geeignet ist, eine Lageänderung des Blickfeldes des optischen Sensors im Raum zu erfassen. Der optische Sensor kann Teil eines TOF-Kamerasystems sein. Der Lagesensor weist zumindest zwei Bewegungsaufnehmer auf, nämlich Beschleunigungs- bzw. Drehratensensoren. Aus den Ausgangssignalen der Beschleunigungs- bzw. Drehratensensoren werden Bewegungen des Instruments im Raum erfasst, so dass auf Grundlage einer definierten Ausgangslage auf eine neue absolute Lage im Raum geschlossen werden kann.

Es ist Aufgabe der vorliegenden Erfindung, eine Vorrichtung zur endoskopischen 3D-Datenerfassung anzugeben, wobei die genannten Nachteile möglichst vermieden werden. Insbesondere ist es Aufgabe der vorliegenden Erfindung, eine Vorrichtung zur endoskopischen 3D-Datenerfassung anzugeben, die einfach handhabbar und vielseitig einsetzbar ist und die die Erfassung der dreidimensionalen Daten mit einer hohen räumlichen Auflösung ermöglicht.

Diese Aufgabe wird durch eine Vorrichtung gemäß Anspruch 1 gelöst. Eine erfindungsgemäße Vorrichtung zur endoskopischen 3D-Datenerfassung, insbesondere zur dreidimensionalen Erfassung einer Oberfläche eines körperinneren Hohlraums, umfasst Lichterzeugungsmittel zur Erzeugung mindestens einer modulierten Messstrahlung. Zur Erzeugung der Messstrahlung können dabei beispielsweise lichtemittierende Dioden (LEDs), Superlumineszenzdioden, Laser, etwa Laserdioden oder Superkontinuum-Laser, oder andere entsprechend modulierbare Strahlungsquellen eingesetzt werden. Laserdioden haben dabei insbesondere den Vorteil einer einfachen Handhabung und sind kostengünstig, kompakt und leicht modulierbar. Dabei haben multimodale Laserdioden in der Regel den Vorteil einer höheren Ausgangsleistung als monomodale Laserdioden. Die Messstrahlung kann sinusförmig modulierbar sein, ggf. über einem Grundniveau. Zur besseren Handhabung und effektiveren Kühlung können die Lichterzeugungsmittel in einem eigenen Gehäuse bzw. als separate Lichtquelle angeordnet sein.

Weiterhin umfasst die erfindungsgemäße Vorrichtung Lichtweiterleitungsmittel zur Weiterleitung der Messstrahlung auf zumindest einen Teilbereich der Oberfläche des körperinneren Hohlraums. Die Lichtweiterleitungsmittel können dabei insbesondere Mittel zur Einkopplung der von der Lichtquelle erzeugten Strahlung in einen Lichtleiter umfassen sowie Lichtleiter zur Weiterleitung der Strahlung. So kann beispielsweise eine Linsen- und/oder Spiegelanordnung zur verbesserten Einkopplung der erzeugten Strahlung vorgesehen sein, oder es können auch fasergekoppelte Superlumineszenz- oder Laserdioden verwendet werden. Sind die Lichterzeugungsmittel in einem eigenen Gehäuse bzw. als separate Lichtquelle angeordnet, kann ein Lichtleitkabel zur Weiterleitung der Strahlung vorgesehen sein, das mit Verbindungsmitteln zur Verbindung mit der Lichtquelle oder weiteren Lichtleitern versehen sein kann.

Die Lichtweiterleitungsmittel sind zumindest teilweise in einem endoskopisch einsetzbaren Schaft angeordnet. Der Schaft ist insbesondere langerstreckt und mit einer solchen Länge und einem solchen Durchmesser ausgebildet, dass er in einem körperinneren Hohlraum durch eine natürliche oder künstlich geschaffene Öffnung einführbar ist. Der Schaft weist ein distales (benutzerfernes) und ein proximales (benutzernahes) Ende auf. Der Schaft kann insbesondere als Teil eines Endoskops ausgebildet sein, das weiterhin einen am proximalen Ende des Schafts angeordneten Endoskopkopf umfasst, wobei am Endoskopkopf beispielsweise ein Anschluss für das Lichtleitkabel zur Verbindung mit den Lichterzeugungsmitteln angeordnet ist. Der Schaft kann auch als Katheter ausgebildet sein. Die Lichtweiterleitungsmittel können insbesondere eine in dem endoskopisch einsetzbaren Schaft angeordnete Beleuchtungsoptik umfassen, durch die die Messstrahlung zur Beleuchtung eines zu beobachtenden Bereichs des Hohlraums bis zum distalen Ende des Schafts weitergeleitet wird. Die Beleuchtungsoptik kann etwa als Bündel von Lichtleitfasern oder als Lichtleitstab ausgebildet sein. Ferner kann am distalen Ende der Beleuchtungsoptik eine Aufweitungsoptik, etwa eine Linse oder eine Streuscheibe, zur gleichmäßigen Verteilung der Messstrahlung auf den zu beobachtenden Bereich angeordnet sein. Zur Vermeidung der Einkopplung unerwünschter Strahlung, beispielsweise zur Verringerung der Wärmebelastung bei einer endoskopischen Anwendung in einem lebenden Körper, können weiterhin Filtermittel vorgesehen sein, die bestimmte Anteile der erzeugten Strahlung ganz oder teilweise wegfiltern.

Die erfindungsgemäße Vorrichtung weist ferner eine in einem distalen Endbereich des Schafts angeordnete Beobachtungsoptik auf zur Aufnahme einer Signalstrahlung von zumindest dem Teilbereich der Oberfläche des Hohlraums. Die Signalstrahlung entsteht insbesondere durch Reflexion und/oder Streuung der modulierten Messstrahlung an der Oberfläche des Hohlraums, kann aber auch andere Anteile umfassen, etwa reflektiertes bzw. gestreutes Weißlicht oder Fluoreszenzstrahlung. Die Beobachtungsoptik weist hierfür insbesondere eine Linsenanordnung auf, beispielsweise ein Endoskopobjektiv, das ein Bild bzw. ein erstes Zwischenbild des Teilbereichs der Oberfläche des Hohlraums erzeugt.

Weiterhin umfasst die erfindungsgemäße Vorrichtung einen zumindest teilweise innerhalb des Schafts angeordneten Bildweiterleiter zum Weiterleiten der Signalstrahlung vom distalen zum proximalen Endbereich des Schafts zur Aufnahme durch einen Time-of-Flight-Bildsensor (Lichtlaufzeit-, TOF-Bildsensor). Ein TOF-Bildsensor ist ein phasenempfindlicher Bildsensor, der insbesondere als ein phasenempfindlich ansteuerbarer Festkörpersensor ausgebildet ist und der eine Mehrzahl von Pixeln umfasst, die pixelweise Lichtlaufzeitinformationen und somit räumlich aufgelöste Abstands- bzw. Tiefendaten von Messpunkten auf der Oberfläche des Hohlraums liefern. Zur Erzeugung eines Bilds des zu beobachtenden Teilbereichs der Oberfläche des körperinneren Hohlraums auf der Sensorfläche des TOF-Bildsensors können Abbildungsmittel, etwa ein Linsensystem, vorgesehen sein. Die Beobachtungsoptik, der Bildweiterleiter und/oder die Abbildungsmittel können Filtermittel zum Abblocken eines Teils der aufgenommenen Strahlung umfassen.

Ferner sind Steuerungs- und Auswertungsmittel vorgesehen zur Ansteuerung der Lichterzeugungsmittel zur Erzeugung der modulierten Messstrahlung, zur Ansteuerung des TOF-Bildsensors und zur Auswertung der von dem TOF-Bildsensor gelieferten Daten zur Erzeugung von 3D-Daten des Teilbereichs der Oberfläche des Hohlraums. Insbesondere ermöglichen die Steuerungsmittel die Erzeugung der Modulation der Messstrahlung und eine entsprechende Ansteuerung des TOF-Bildsensors zur phasenselektiven Aufnahme der empfangenen Strahlung und zum Auslesen des Signals des TOF-Bildsensors, das pixelweise Phaseninformationen in Bezug auf die Modulation der Signalstrahlung enthält. Aus den Phaseninformationen kann auf die Zeitverzögerung zwischen dem Auftreffen der Signalstrahlung und der Aussendung der Messstrahlung geschlossen werden, wodurch pixelweise Tiefeninformationen gewonnen werden können. Weiterhin kann eine Anzeigeeinrichtung zur Anzeige der aufgenommenen Abstandsinformationen bzw. der 3D-Daten vorgesehen sein.

Erfindungsgemäß umfasst die Vorrichtung weiterhin Lagesensormittel zur Erfassung einer Position und einer Orientierung des Schafts. Insbesondere sind die Lagesensormittel innerhalb des Schafts angeordnet oder diesem in einer eindeutigen geometrischen Beziehung zugeordnet. Die Lagesensormittel sind zum Zusammenwirken mit einem Lageerfassungssystem ausgebildet, wodurch die Position und die Orientierung des Schafts relativ zu einem absoluten, von der Lage des Schafts unabhängigen, extrakorporalen Referenzkoordinatensystem bestimmt werden können. Aufgrund der bekannten Zuordnung der Lagesensormittel zu einem distalen Endbereich des Schafts kann aus den Signalen der Lagesensormittel auf die Position und die Orientierung des distalen Endbereichs des Schafts bzw. der darin angeordneten Beobachtungsoptik in Bezug zu dem Referenzkoordinatensystem geschlossen werden. Die Steuerungs- und Auswertungsmittel sind dazu ausgebildet, aufgrund der mit Hilfe der Lagesensormittel gewonnenen Daten aus den vom TOF-Bildsensor gelieferten räumlich aufgelösten Tiefeninformationen 3D-Daten zu berechnen, die die Oberfläche des körperinneren Hohlraums in Bezug zu dem Referenzkoordinatensystem dreidimensional darstellen.

Durch den TOF-Bildsensor ist eine zweidimensional aufgelöste Erfassung von Abstands- bzw. Tiefeninformationen der Oberfläche des Hohlraums mit einer hohen Auflösung möglich. Dadurch, dass Lagesensormittel vorgesehen sind, sind Informationen zur Position und Orientierung des endoskopisch einsetzbaren Schafts erfassbar, die zusammen mit den vom TOF-Bildsensor gelieferten Daten die Ermittlung absoluter Koordinaten von Punkten auf der Oberfläche des Hohlraums gestatten. Hierdurch wird auf besonders einfache Weise eine dreidimensionale Erfassung bzw. räumliche Rekonstruktion zumindest eines Teilbereichs einer Oberfläche eines körperinneren Hohlraums ermöglicht. Insbesondere können absolute Organkoordinaten ermittelt werden, die beispielsweise die Vermessung intrakorporaler Strukturen ermöglichen, etwa die Bestimmung der Größe eines Tumors oder die flächenhafte Vermessung von Läsionen. Ferner ist hierdurch auf besonders einfache Weise die Erstellung eines virtuellen 3D-Modells der Oberfläche des körperinneren Hohlraums möglich. Diese Daten können zum Beispiel mit 3D-Oberflächendaten korreliert werden, die etwa über CT- oder MR-Bildgebungssysteme präoperativ gewonnen worden sind. Dadurch wird auch eine einfachere Orientierung für einen Benutzer, etwa einen Chirurgen, bei einer endoskopischen Untersuchung oder einem endoskopischen Eingriff innerhalb des körperinneren Hohlraums ermöglicht. Bei endoskopischen Eingriffen über einen intrakorporalen Zugang (NOTES) wird eine einfachere Orientierung innerhalb des Hohlraums ermöglicht, in dem der endoskopische Zugang erfolgt. Bei der extrakorporalen Lithotripsie können Steine endoskopisch über eine Sichtdistanz geortet und die extrakorporale Stoßwellen-Quelle sicherer ausgerichtet werden, ohne dass in jedem Fall der Einsatz eines Röntgengeräts notwendig wäre; der Verlauf der Lithotripsie kann außerdem bei Einhaltung einer entsprechenden Sichtdistanz zum Schutz des Endoskops vor den Auswirkungen der Stoßwellen endoskopisch kontrolliert werden. Ebenso können durch begleitende Verwendung der erfindungsgemäßen Vorrichtung Tumorbestrahlungen sicherer ausgeführt werden, indem die Lage des zu bestrahlenden Tumors fortlaufend erfasst und visuell kontrolliert wird und die Position des Tumors an das Bestrahlungsgerät zurückgemeldet wird. Dadurch ist auch eine Bestrahlung von "lagevarianten" Tumoren an leicht beweglichen Hohlorganen wie zum Beispiel im Darm oder in der Blase möglich. Insbesondere bei einer dünnkalibrigen Ausführung des Schafts ist ein Einsatz im Dentalbereich sowie in der Vaskularendoskopie möglich, etwa für eine dreidimensionale Rekonstruktion des Lumens eines dentalen Wurzelkanals sowie für eine intraluminale Vermessung von Stenosen.

Gemäß einer bevorzugten Ausführungsform der Erfindung ist der Schaft starr ausgebildet. In dem Fall, dass der endoskopisch einführbare Schaft Teil eines Endoskops ist, kann das Endoskop dementsprechend nach Art eines starren Endoskops ausgebildet sein. Der Endoskopschaft kann zumindest teilweise als zylindrisches Rohr, insbesondere metallisches Rohr, ausgebildet sein. Der Bildweiterleiter kann in diesem Fall durch ein oder mehrere innerhalb des Schafts angeordnete Relais-Linsensysteme gebildet werden. Die Lagesensormittel können an einem beliebigen Ort im Schaft angeordnet sein; ist der Schaft Teil eines Endoskops, können die Lagesensormittel auch im oder am Kopf des Endoskops angeordnet sein. Da der Abstand und die relative Orientierung des distalen Endbereichs des Schafts im Verhältnis zu den Lagesensormitteln bekannt sind, kann aus den von einem Lageerfassungssystem gelieferten Daten über die Position und Orientierung der Lagesensormittel auf die Position und Orientierung des distalen Endbereichs des Schafts und damit der Beobachtungsoptik geschlossen werden. Da ferner die Lichtlaufzeit von der Lichterzeugungseinrichtung zum distalen Endbereich des Schafts sowie von der Beobachtungsoptik zum TOF-Bildsensor bekannt ist, können somit die von dem TOF-Bildsensor gelieferten Abstandsdaten in absolute Koordinaten von Messpunkten auf der Oberfläche des Hohlraums umgerechnet werden, wobei der TOF-Bildsensor eine hohe räumliche Auflösung ermöglicht.

Gemäß einer anderen bevorzugten Ausführungsform der Erfindung ist der Schaft flexibel ausgebildet. In dem Fall, dass der endoskopisch einführbare Schaft Teil eines Endoskops ist, kann das Endoskop dementsprechend nach Art eines flexiblen Endoskops ausgebildet sein. Auch ein Katheter wird im Rahmen der vorliegenden Anmeldung als flexibler Schaft verstanden. Das flexible Endoskop kann insbesondere steuerbar sein, d.h., der distale Endbereich des Schafts kann durch am proximalen Endbereich des Endoskops angeordnete Bedienelemente abgewinkelt werden. Der Schaft kann auch halbstarr bzw. das Endoskop als halbstarres Endoskop ausgebildet sein. Bei einem flexiblen oder einem halbstarren Schaft ist der Bildweiterleiter zumindest innerhalb des Schafts als flexibler Bildleiter, der etwa durch ein geordnetes Glasfaserbündel gebildet sein kann, ausgebildet. Der Bildleiter weist bevorzugt einen kleinen Durchmesser auf, vorzugsweise unter 1,5 mm, weiter bevorzugt unter 1,0 mm oder sogar unter 0,6 mm. Der Bildleiter kann bevorzugt eine Länge von über 150 cm, sogar über 300 cm aufweisen. Vorzugsweise ist der Bildleiter in Zweiglastechnik als Multifaserbündel hergestellt. Die Lagesensormittel sind bei dieser Ausgestaltung der Erfindung im distalen Endbereich des Schafts angeordnet. Aufgrund des von einem Lageerfassungssystem gelieferten Signals kann die Position und die Orientierung des distalen Endbereichs des Schafts in dem durch das Lageerfassungssystem gegebenen absoluten Koordinatensystem ermittelt werden. Hieraus können mit den vom TOF-Bildsensor gelieferten Tiefeninformationen absolute Koordinaten von Messpunkten auf der Oberfläche des körperinneren Hohlraums mit hoher räumlicher Auflösung berechnet werden.

Vorzugsweise umfassen die Lagesensormittel einen Positionssensor, wodurch eine Position in Bezug auf ein extrakorporales Referenzsystem ermittelt werden kann. Im Unterschied etwa zu einem Trägheits- bzw. Inertialsensor ermöglicht ein Positionssensor eine unmittelbare Erfassung einer Position relativ zu einem Referenzkoordinatensystem. Demgegenüber erfasst ein Inertialsensor eine Beschleunigung, aus der eine Position mittelbar durch zweifache zeitliche Integration ermittelt wird. Inertiale Lagesensoren unterliegen daher einer zeitlichen Drift, so dass die Ungenauigkeit der ermittelten Position mit zunehmender Zeit immer größer wird. Die Lagesensormittel können in vorteilhafter Weise als Positions- und Orientierungssensor ausgebildet sein, der eine unmittelbare Erfassung einer Position und einer räumlichen Orientierung in Bezug zu einem Referenzsystem ermöglicht. Inertiale Lagesensoren ermöglichen demgegenüber in der Regel lediglich die unmittelbare Erfassung einer Ausrichtung des Sensors relativ zu einer durch die Schwerkraft gegebenen Richtung, während insbesondere die Orientierung in einer horizontalen Ebene mittelbar durch zeitliche Integration bestimmt wird, was ebenfalls eine zeitliche Drift zur Folge haben kann. Dadurch, dass die Lagesensormittel einen nicht-inertialen Sensor zur Ermittlung einer Position bzw. Orientierung des Endoskops, insbesondere des distalen Endbereichs des Endoskopschafts, umfassen, ist auch bei einem langdauernden endoskopischen Eingriff eine hohe Genauigkeit hinsichtlich der Position und räumlichen Orientierung relativ zu einem extrakorporalen Referenzkoordinatensystem erreichbar.

Die Lagesensormittel sind erfindungsgemäß als elektromagnetischer Lagesensor ausgebildet. Ein solcher elektromagnetischer Lagesensor wirkt insbesondere derart mit einem von einem externen Lageerfassungssystem erzeugten Magnetfeld zusammen, dass eine unmittelbare, d.h. keine zweifache zeitliche Integration erfordernde Bestimmung einer Position und/oder Orientierung in Bezug auf ein durch das externe Lageerfassungssystem gegebenes Referenzkoordinatensystem möglich ist. Dabei umfasst der elektromagnetische Lagesensor erfindungsgemäß mindestens zwei Spulen, auf die jeweils Spannungen durch ein extrakorporales magnetfelderzeugendes Element des externen Lageerfassungssystems induziert werden. Der elektromagnetische Lagesensor wirkt derart mit dem von dem externen Lageerfassungssystem erzeugten Magnetfeld zusammen, dass aus einem von den Spulen gelieferten Strom- bzw. Spannungssignal die Lage jeder der beiden Spulen relativ zu dem Lageerfassungssystem ermittelbar ist. Auf diese Weise können absolute, d.h. auf das Lageerfassungssystem bezogene, Koordinaten des elektromagnetischen Lagesensors bestimmt und die räumliche Orientierung des Lagesensors mit einer auch über die Dauer einer langen Operation ausreichenden Genauigkeit ermittelt werden. Es können auch mehrere derartige Lagesensoren mit jeweils mindestens zwei Spulen vorhanden sein.

Gemäß einer bevorzugten Ausführungsform der Erfindung umgeben die mindestens zwei Spulen des elektromagnetischen Lagesensors den Bildweiterleiter. Insbesondere umgeben die mindestens zwei Spulen den Bildleiter seitlich in einem distalseitigen Endbereich des Bildleiters, wobei die Spulen vorzugsweise in einer Längsrichtung des Schafts zueinander versetzt sind. In vorteilhafter Weise können zwei Spulen in Längsrichtung voneinander beabstandet koaxial den Bildleiter umgeben; die Spulen können auf einem metallischen Träger angeordnet sein bzw. einen Eisenkern aufweisen. Um eine Beeinträchtigung der Funktion des elektromagnetischen Lagesensors zu vermeiden, ist in vorteilhafter Weise der Schaft zumindest in dem Bereich, in dem der Lagesensor angeordnet ist, nichtmetallisch, beispielsweise aus Kunststoff oder Keramik, ausgebildet; das gleiche gilt für den Kopf eines starren Endoskops, wenn in diesem der Lagesensor angeordnet ist.

Insbesondere können die beiden Spulen innerhalb eines starren Schafts oder im distalen Endbereich eines flexiblen Schafts um den Bildleiter gewickelt sein, um die Erfassung der Position und Orientierung des distalen Endbereichs des Schafts zu ermöglichen. Hierdurch wird zudem eine platzsparende Anordnung geschaffen, die eine Aufnahme der Lagesensormittel im Schaft des Endoskops, insbesondere im distalen Endbereich des Schafts, erlaubt, ohne dass der Durchmesser des Schafts hierdurch wesentlich vergrößert wird. Dies ermöglicht eine besonders dünnkalibrige Ausführung des Schafts bzw. des Endoskops.

Vorzugsweise umfasst das externe Lageerfassungssystem einen extrakorporalen Magnetfeldgenerator, der mittels tetraedrisch angeordneter Spulen ein inhomogenes Magnetfeld erzeugt, über das der mindestens eine elektromagnetische Lagesensor anregbar ist. Ein derartiger Magnetfeldgenerator wird von der Firma NDI EUROPE GmbH unter dem Handelsnamen AURORA vertrieben. Es sind allerdings auch flachbauende Spulenanordnungen verfügbar, die sich aufgrund ihrer kompakten Ausgestaltung insbesondere für die Anwendung in der Chirurgie eignen. Das externe Lageerfassungssystem umfasst weiterhin vorzugsweise eine Auswertevorrichtung, die die von dem zumindest einen Lagesensor erzeugten Signale hinsichtlich der Position und/oder Orientierung des Lagesensors und somit des Schafts in Korrelation zu einer räumlichen Referenz auswertet. Die Auswertevorrichtung ermöglicht es, die von dem Lagesensor erzeugten Signale in eine Lageinformation zu verarbeiten, die vorteilhafterweise zur Navigation des Schafts bzw. eines Endoskops beispielsweise bei einem chirurgischen Einsatz genutzt werden kann.

Alternativ oder zusätzlich kann mindestens ein Lagesensor vorgesehen sein, der als Inertialsensor ausgebildet ist und durch Messung von Trägheitskräften eine indirekte Ermittlung einer aktuellen Position und Orientierung des Lagesensors ermöglicht. Ferner kann eine Lageerfassung mittels Ultraschall vorgesehen sein. Bei einer starren Ausbildung des Schafts ist auch eine Lageerfassung durch ein optisches Trackersystem möglich; ein solches wird beispielsweise von KARL STORZ unter der Bezeichnung SURGICAL COCKPIT® Navigation Panel Unit angeboten.

Erfindungsgemäß ist der TOF-Bildsensor über einen flexiblen Bildleiter mit dem proximalen Endbereich des Bildweiterleiters des Schafts lösbar verbindbar. Ist der Bildweiterleiter innerhalb des Schafts mit einem oder mehreren Relaislinsensystemen ausgebildet, so ist der flexible Bildleiter des TOF-Bildsensors an den Schaft derart ansetzbar, dass das Relaislinsensystem auf der Eingangsfläche des flexiblen Bildleiters ein Zwischenbild erzeugt. Ist der Bildweiterleiter innerhalb des Schafts als flexibler Bildleiter ausgebildet, so kann ein weiterer flexibler Bildleiter an eine proximale Endfläche des im Schaft geführten Bildleiters ansetzbar sein; hierfür kann eine Bildleiterkupplung vorgesehen sein. In dem Fall, dass der Bildweiterleiter innerhalb des Schafts als flexibler Bildleiter ausgebildet ist, kann dieser aber auch durchgehend bis zum TOF-Bildsensor ausgebildet und hierzu über den Schaft hinaus in proximaler Richtung weitergeführt sein. Zur optimalen optischen Ankopplung des TOF-Bildsensors an den Bildleiter kann eine Abbildungsoptik vorgesehen sein, die das vom Bildleiter gelieferte Bild auf das Format des TOF-Bildsensors vergrößert.

Dadurch, dass der TOF-Bildsensor über einen flexiblen Bildleiter mit dem proximalen Endbereich des Bildweiterleiters des Schafts verbunden bzw. verbindbar ist, kann trotz des Raumbedarfs von TOF-Bildsensoren, insbesondere von solchen mit höherer Auflösung, der Schaft mit einem besonders kleinen Durchmesser ausgebildet sein; auch das Gewicht des TOF-Bildsensors ist dann unkritisch. Der Schaft bzw. das Endoskop kann besonders handlich ausgebildet sein, da der TOF-Bildsensor nicht Teil des Schafts bzw. des Endoskops ist und mit diesem nicht starr verbunden ist. Auf diese Weise ist eine besonders hohe Auflösung der von dem TOF-Bildsensor gelieferten 3D-Daten erzielbar, wobei die Vorrichtung bzw. das Endoskop dennoch besonders einfach handhabbar und besonders vielseitig einsetzbar ist. Dabei ist zu berücksichtigen, dass TOF-Sensoren häufig im infraroten Spektralbereich arbeiten und die Pixelgröße derartiger Sensoren in der Regel größer ist als die von im visuellen Bereich arbeitenden Standard-Bildsensoren. Insbesondere ist eine derartige Vorrichtung bzw. ein derartiges Endoskop auch in Fällen einsetzbar, in denen der Durchmesser des Schafts aus anatomischen Gründen auf wenige Millimeter oder sogar auf weniger als 1 mm beschränkt ist. Ferner hat die vom Schaft bzw. vom Endoskop separate Ausführung des TOF-Bildsensors den Vorteil, dass dieser beim Einsatz der Vorrichtung patientenfern angeordnet sein kann und deshalb hinsichtlich Sicherheit, Reinigung und Sterilisierung nicht die gleichen Anforderungen erfüllen muss wie das Endoskop selbst.

Weiterhin erfindungsgemäß sind im distalen Endbereich des Schafts ein Strahlteiler und ein weiterer Bildsensor zur Erfassung eines weiteren Bilds des beobachteten Teilbereichs der Oberfläche des Hohlraums angeordnet. Während der TOF-Bildsensor zur Ermittlung der Abstandsdaten ein Bild im infraroten Spektralbereich aufnimmt, ist das weitere Bild, das von dem weiteren Bildsensor aufgenommen wird, ein visuelles Bild des Teilbereichs der Oberfläche. Der distale Strahlteiler ist hierfür derart angeordnet, dass ein Teil der von der Beobachtungsoptik aufgenommenen Signalstrahlung auf die Oberfläche des weiteren Bildsensors geleitet wird, während ein anderer Teil der Signalstrahlung in den Bildweiterleiter zur Weiterleitung zum TOF-Bildsensor eingekoppelt wird. Der weitere Bildsensor ist in vorteilhafter Weise kompakt ausgebildet, so dass der Durchmesser des Schafts innerhalb des zur Einführung in einen körperinneren Hohlraum zulässigen Bereichs bleibt. Zur Versorgung des weiteren Bildsensors sowie zur Weiterleitung der von diesem aufgenommenen Bilddaten können im Schaft neben dem Bildweiterleiter eine oder mehrere elektrische Leitungen angeordnet sein. Durch die Aufnahme des weiteren Bilds wird eine vollständigere Erfassung und Darstellung des beobachteten Teilbereichs der Oberfläche des Hohlraums und die visuelle Gewinnung weiterer Informationen ermöglicht.

Weiterhin ist es bevorzugt, dass eine Bildverarbeitungseinrichtung vorgesehen ist, die eingerichtet ist, um einer Mehrzahl von Bildpunkten, insbesondere jedem Bildpunkt des von dem weiteren Bildsensor aufgenommenen Bilds jeweils eine Tiefeninformation zuzuordnen, die aus der Bildinformation des TOF-Bildsensors gewonnen wird. Sofern der TOF-Bildsensor eine andere Auflösung als der weitere Bildsensor hat, kann durch die Bildverarbeitungseinrichtung beispielsweise eine entsprechende Interpolation zwischen benachbarten Pixeln vorgenommen werden. Die Bildverarbeitungseinrichtung kann auch dazu ausgebildet sein, die Bilddaten des TOF-Bildsensors zu glätten, indem beispielsweise Abstandswerte von Pixeln, die vom Mittelwert der Abstandswerte jeweils benachbarter Pixel um mehr als einen Schwellwert abweichen, durch den Mittelwert ersetzt werden. Hierdurch kann ein von dem weiteren Bildsensor aufgenommenes Endoskopiebild mit den Abstandsdaten bzw. mit einem erstellten virtuellen 3D-Modell des körperinneren Hohlraums verknüpft und ggf. gemeinsam mit dem virtuellen 3D-Modell auf einer Anzeigeeinrichtung dargestellt werden.

Gemäß einer Ausführungsform der Erfindung ist mit dem proximalen Endbereich des Bildweiterleiters ein Strahlteiler verbunden oder lösbar verbindbar, der einen Teil der von dem Bildweiterleiter übertragenen Strahlung auskoppelt. Die ausgekoppelte Strahlung kann insbesondere weiteren Bildsensoren zugeführt werden, wodurch weitere Bilddaten erzeugt werden können. Vorzugsweise wird die ausgekoppelte Strahlung Spektralanalysemitteln, beispielsweise zur Raman- bzw. CARS-Spektroskopie, zugeführt, wodurch Spektraldaten erzeugt werden können. Besonders vorteilhaft ist es, wenn ein distal angeordneter Strahlteiler auch einen Teil des visuellen Spektralbereichs durchlässt, so dass dieser über den Bildweiterleiter zum proximal angeordneten Strahlteiler gelangt und dort für die Spektralanalyse ausgekoppelt wird. Hierdurch werden weitere Möglichkeiten zur Darstellung der endoskopischen Szene und/oder für endoskopische Diagnosen eröffnet.

Gemäß einer weiteren bevorzugten Ausführungsform der Erfindung ist der Bildweiterleiter auch zum Weiterleiten einer Fluoreszenzanregungsstrahlung zum distalen Endbereich des Schafts ausgebildet, von wo die Fluoreszenzanregungsstrahlung auf den Teilbereich der Oberfläche des körperinneren Hohlraums geleitet wird. Die Fluoreszenzanregungsstrahlung kann beispielsweise im blauen Spektralbereich des visuellen Spektrums liegen, während die modulierte Messstrahlung in der Regel im infraroten Spektralbereich liegt. Die Fluoreszenzanregungsstrahlung kann direkt oder über einen vorzugsweise spektral selektiven Strahlteiler in den proximalen Endbereich des Bildweiterleiters eingekoppelt werden. Der im distalen Endbereich des Schafts angeordnete Strahlteiler und ein dort angeordneter weiterer Bildsensor können in diesem Fall insbesondere zur Erfassung der Fluoreszenzstrahlung, die vom beobachteten Teilbereich der Oberfläche des Hohlraums emittiert wird, ausgebildet sein.

Ein nicht von der Erfindung umfasstes Verfahren zur endoskopischen 3D-Datenerfassung umfasst zumindest die folgenden Schritte:
- Erzeugen mindestens einer modulierten Messstrahlung,
- Weiterleiten der modulierten Messstrahlung auf zumindest einen Teilbereich einer Oberfläche eines körperinneren Hohlraums durch einen endoskopisch einsetzbaren Schaft, der beispielsweise Teil eines Endoskops sein kann,
- Aufnehmen einer Signalstrahlung von zumindest dem Teilbereich der Oberfläche des Hohlraums mit Hilfe einer in einem distalen Endbereich des Schafts angeordneten Beobachtungsoptik,
- Weiterleiten der Signalstrahlung vom distalen zu einem proximalen Endbereich des Schafts mit Hilfe eines zumindest teilweise innerhalb des Schafts angeordneten Bildweiterleiters,
- Aufnehmen der Signalstrahlung durch einen Time-of-Flight-Bildsensor (TOF-Bildsensor)
- Auswertung der von dem TOF-Bildsensor gelieferten Daten zur Erzeugung von auf die Beobachtungsoptik bzw. den distalen Endbereich des Schafts bezogenen 3D-Daten des Teilbereichs der Oberfläche des Hohlraums,
- Ermittlung von Informationen zur Position und Orientierung des Schafts mit Hilfe von Lagesensormitteln und
- Berechnung absoluter 3D-Daten des Teilbereichs der Oberfläche des Hohlraums aufgrund der auf die Beobachtungsoptik bzw. den distalen Endbereich des Schafts bezogenen 3D-Daten und der mit Hilfe der Lagesensormittel ermittelten Informationen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind.

Weitere Aspekte der Erfindung ergeben sich aus der nachfolgenden Beschreibung eines bevorzugten Ausführungsbeispiels und der beigefügten Zeichnung. Es zeigen:
Fig. 1 ein Endoskop als Teil einer Vorrichtung zur endoskopischen Datenerfassung im schematischen Längsschnitt gemäß einem ersten Ausführungsbeispiel;
Fig. 2a eine Beobachtungsoptik und einen Bildweiterleiter gemäß einem zweiten Ausführungsbeispiel einer Vorrichtung in schematischer Darstellung;
Fig. 2b die Beobachtungsoptik gemäß dem Ausführungsbeispiel der Fig. 2a in vergrößerter schematischer Darstellung;
Fig. 3 ein Endoskop als Teil einer Vorrichtung zur endoskopischen Datenerfassung im schematischen Längsschnitt gemäß einem dritten Ausführungsbeispiel;
Fig. 4 ein Endoskop als Teil einer Vorrichtung zur endoskopischen Datenerfassung im schematischen Längsschnitt gemäß einem vierten Ausführungsbeispiel;
Fig. 5a eine erfindungsgemäße Vorrichtung gemäß einem Ausführungsbeispiel der Erfindung in schematischer Darstellung;
Fig. 5b den distalen Endbereich des Endoskopschafts der Vorrichtung der Fig. 5a im schematischen Längsschnitt;
Fig. 5c eine Versorgungseinheit der Vorrichtung der Fig. 5a in schematischer Darstellung.

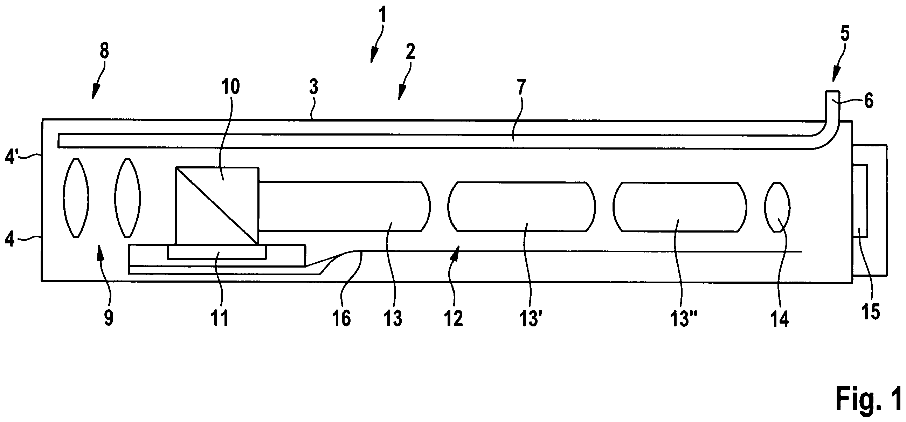

Wie in Fig. 1 schematisch gezeigt, umfasst ein Endoskop 1 gemäß einem Ausführungsbeispiel einen langerstreckten starren Schaft 2, der durch ein zylindrisches Rohr 3 gebildet wird, das ein oder mehrere distale Fenster 4, 4' aufweist und in dem weitere optische, mechanische und elektronische Komponenten aufgenommen sind. Im proximalen Endbereich 5 des Schafts, der als Endoskopkopf ausgebildet ist, ist ein Lichtanschlussstutzen 6 angeordnet, an den ein Lichtleitkabel zur Verbindung mit einer nicht dargestellten Lichtquelle angekoppelt werden kann. Die Lichtquelle erzeugt sowohl eine modulierte Messstrahlung für eine Abstandsmessung als auch ein Beleuchtungslicht zur visuellen Beobachtung eines Oberflächenbereichs eines körperinneren Hohlraums. Die Messstrahlung und das Beleuchtungslicht werden hier als "Licht" bezeichnet, unabhängig davon, ob es sich um sichtbares Licht oder um infrarote oder ultraviolette Strahlung handelt. Innerhalb des Schafts 2 ist ein Lichtleiter 7, der beispielsweise aus einem Glasfaserbündel bestehen kann, zur Weiterleitung des eingekoppelten Lichts zum distalen Endbereich 8 des Endoskops angeordnet; es kann auch eine Mehrzahl von Lichtleitern vorgesehen sein. Am distalen Ende des Lichtleiters 7 kann eine Aufweitungsoptik zur gleichmäßigen Ausleuchtung eines zu beobachtenden Bereichs vorgesehen sein (nicht dargestellt). Durch das Fenster 4' tritt das vom Lichtleiter 7 weitergeleitete Licht in Richtung auf den zu beobachtenden Bereich des Hohlraums aus.

Von dem beobachteten Bereich der Oberfläche des Hohlraums tritt eine Signalstrahlung durch das Fenster 4, das mit dem Fenster 4' einstückig ausgebildet sein kann, in die Beobachtungsoptik des Endoskops 1 ein, die insbesondere als Endoskopobjektiv 9 ausgebildet ist. Die Signalstrahlung umfasst einen Anteil, der von der Messstrahlung durch Reflexion an der Oberfläche des Hohlraums und/oder durch Streuung erzeugt wird. Ferner tritt das von der Oberfläche reflektierte visuelle Beleuchtungslicht sowie ggf. Fluoreszenzlicht in die Beobachtungsoptik ein. Im Beobachtungsstrahlengang ist nach dem Endoskopobjektiv 9 ein Strahlteiler 10 angeordnet, der einen Anteil des aufgenommenen Lichts quer zur Längsrichtung des Schafts 2 zu einem im distalen Endbereich 8 des Endoskops 1 angeordneten Bildsensor 11 ablenkt. Die optische Achse des Bildsensors 11 ist näherungsweise senkrecht zur Schaftlängsrichtung gerichtet, d.h. die Fläche des Bildsensors 11 ist in der Schaftlängsrichtung angeordnet. Ein weiterer Anteil des aufgenommenen Lichts, der zumindest einen Anteil der Signalstrahlung enthält, beispielsweise ein Nahinfrarotanteil, wird vom Strahlteiler 10 in Längsrichtung des Schafts 2 zu einem Bildweiterleiter 12 durchgelassen, der Stablinsen 13, 13', 13" bzw. von diesen gebildete Relaislinsensysteme umfasst. Der Strahlengang ist bildseitig telezentrisch, um eine möglichst effiziente Einkopplung in die Stablinse 13 zu erreichen. Ferner ist die Apertur des vom Objektiv 9 erzeugten Strahlenbündels an die Apertur der Stablinsen 13, 13', 13" angepasst, um diese möglichst auszufüllen. Das vom Objektiv 9, das insbesondere ein Retrofokus-Objektiv ist, erzeugte Bild liegt auf der proximalseitigen Austrittsfläche des Strahlteilers 10, ebenso wie auf der seitlichen, dem Bildsensor 11 zugewandten Austrittsfläche, oder zumindest nahe an diesen Austrittsflächen. Auf die proximalseitige Austrittsfläche des Strahlteilers 10 ist die Stablinse 13 vorzugsweise mit einem Kleber aufgekittet, dessen Brechungsindex gleich dem der Stablinse 13 oder jedenfalls kleiner als der größere von den Brechungsindizes des Strahlteilers 10 und der Stablinse 13 ist.

Über eine Abbildungsoptik 14 wird auf einer Oberfläche eines TOF-Bildsensors 15 ein Bild der Oberfläche des Hohlraums erzeugt. Der Strahlteiler 10 kann beispielsweise derart spektral selektiv ausgebildet sein, dass Licht im visuellen Bereich auf den Bildsensor 11 zur Aufnahme eines visuellen Bilds des beobachteten Bereichs gelenkt wird, während im infraroten Bereich die Signalstrahlung durch den Strahlteiler 10 hindurch gelassen wird und zur Erzeugung räumlich aufgelöster Abstandsdaten auf den TOF-Bildsensor 15 gelangt. Liegt auch die Messstrahlung im visuellen Spektralbereich, so ist andererseits eine nicht spektral selektive Ausbildung des Strahlteilers 10 vorteilhaft.

Zur Versorgung des distalen Bildsensors 11 sowie zur Datenübertragung an die nicht dargestellte Steuerungs- und Auswertungseinrichtung ist eine elektrische Leitung 16 vorgesehen, die ebenfalls innerhalb des Schafts 2 angeordnet ist. Diese kann im proximalen Endbereich 5 des Schafts 2 in einem Stecker zum Anschluss eines entsprechenden Kabels enden oder mit entsprechenden Leitungen des TOF-Bildsensors 15 in einem gemeinsamen Stecker enden bzw. in einem gemeinsamen Kabel geführt werden (nicht dargestellt). Das Endoskop 1 umfasst ferner Lagesensormittel, insbesondere zwei Spulen, die in raumsparender Anordnung den Bildweiterleiter umgeben (nicht dargestellt). Aufgrund der starren Ausbildung des Schafts 2 lässt sich durch Ermittlung der Position und Orientierung der Lagesensormittel die Position und Orientierung des distalen Endbereichs 8 des Schafts 2 ermitteln, insbesondere des Endoskopobjektivs 9 sowie der von diesem erzeugten Bilder des beobachteten Bereichs der Oberfläche des Hohlraums. Hiermit können durch die Steuerungs- und Auswertungseinrichtung aufgrund der vom TOF-Bildsensor 31 erfassten 3D-Daten absolute, auf ein vom Endoskop 1 unabhängiges Koordinatensystem bezogene 3D-Daten berechnet werden.

In dem in Fig. 1 gezeigten Ausführungsbeispiel ist der TOF-Bildsensor 15 mit dem proximalen Ende des endoskopischen Schafts 2 verbunden bzw. in einem proximalen Endbereich 5 des Schafts 2, der als Endoskopkopf ausgebildet ist, aufgenommen. In einem nicht gezeigten Ausführungsbeispiel ist der TOF-Bildsensor über einen flexiblen Bildleiter mit dem proximalen Ende des Schafts 2 verbindbar. Insbesondere dann, wenn der TOF-Bildsensor eine höhere Auflösung und dementsprechend einen größeren Raumbedarf hat, wird hierdurch die Handhabung des Endoskops 1 wesentlich erleichtert. Der visuelle Bildsensor 11 kann andererseits ausreichend kompakt ausgebildet sein, um im distalen Endbereich 8 des Schafts 2 aufgenommen zu sein, ohne eine wesentliche Vergrößerung des Durchmessers des Schafts 2 zu verursachen; auch die elektrische Leitung 16 verursacht keine Vergrößerung des Schaftdurchmessers.

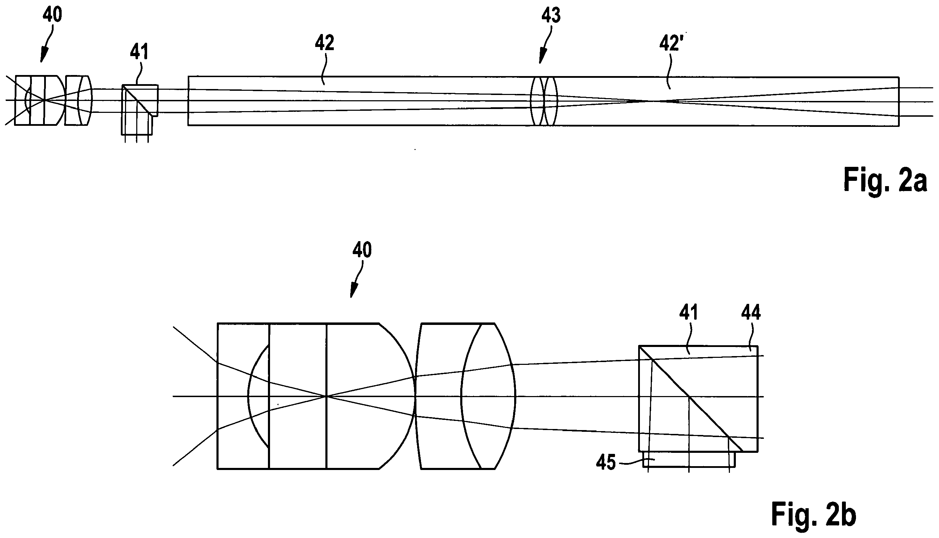

In Fig. 2a sind eine Beobachtungsoptik und ein Bildweiterleiter gemäß einem weiteren Ausführungsbeispiel einer Vorrichtung schematisch gezeigt. Die Beobachtungsoptik umfasst ein Endoskopobjektiv 40, das aus mehreren Linsengruppen besteht. Der Strahlengang ist bildseitig telezentrisch. Im Beobachtungsstrahlengang nach dem Endoskopobjektiv 40 ist ein Strahlteiler 41 angeordnet, der in proximaler Richtung von einem durch Stablinsen 42, 42' gebildeten Relaislinsensystem 43 gefolgt wird. Das Endoskopobjektiv 40 und der Strahlteiler 41 sind in Fig. 2b in vergrößerter Darstellung gezeigt. Wie in Fig. 2b erkennbar ist, kann der Strahlteiler 41 gegenüber der Würfelform in axialer Richtung eine Verlängerung 44 sowie in Querrichtung eine aufgekittete planparallele Platte 45 aufweisen. Durch die planparallele Platte 45 wird erreicht, dass die optischen Weglängen beider optischen Pfade innerhalb des Strahlteilers gleich oder zumindest nahezu gleich sind. Hierdurch wird die Abbildungsqualität verbessert, ferner sind beide Bilder gleich groß. Auch bei den anderen beschriebenen Ausführungsbeispielen sind die optischen Weglängen der beiden Pfade im Strahlteiler idealerweise gleich. Die in Fig. 2a und 2b gezeigte Anordnung kann in einem starren Schaft gemäß Fig. 1 eingesetzt werden.

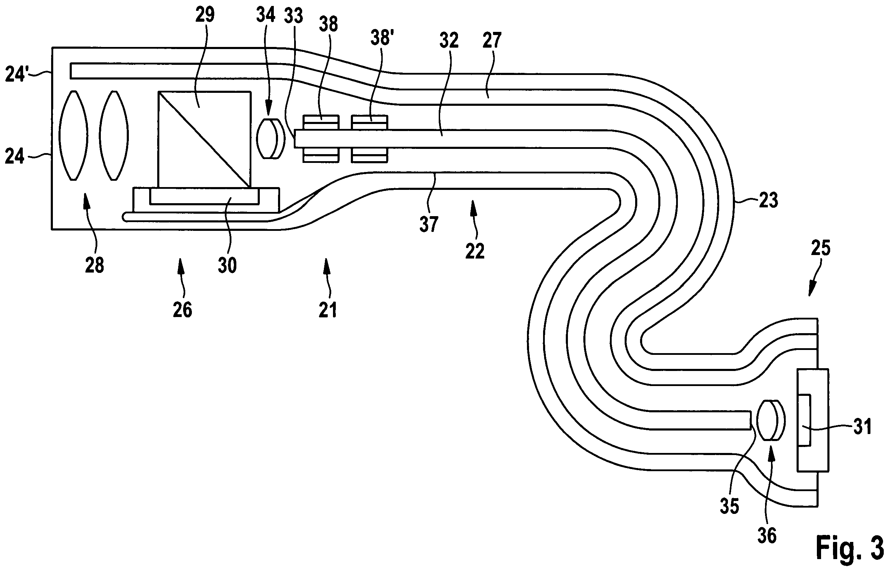

Wie in Fig. 3 gezeigt, umfasst ein Endoskop 21 gemäß einem weiteren Ausführungsbeispiel einen langerstreckten flexiblen Schaft 22. Der Schaft 22 umfasst einen flexiblen Außenschaft 23, der in seinem distalen Endbereich durch ein oder mehrere distale Fenster 24, 24' abgeschlossen ist. Innerhalb des flexiblen Außenschafts sind weitere optische, mechanische und elektronische Komponenten aufgenommen. Im proximalen Endbereich des Schafts ist ein Endoskopkopf 25 angeordnet, der beispielsweise Bedienelemente zur Steuerung der Endoskopspitze, d.h. des distalen Endbereichs 26 sowie Spül- und Sauganschlüsse umfassen kann (nicht dargestellt). Ferner können am Endoskopkopf 25 ein Lichtleitkabel zur Verbindung mit einer Lichtquelle sowie elektrische Versorgungs- und Signalkabel angekoppelt werden (nicht dargestellt).

Wie bereits zu Fig. 1 beschrieben, werden die Messstrahlung und das Beleuchtungslicht durch einen Lichtleiter 27 zum distalen Endbereich 26 des Endoskop 21 geführt und ggf. über eine nicht dargestellte Aufweitungsoptik durch das Fenster 24' auf einen Oberflächenbereich eines körperinneren Hohlraums geleitet. Der Lichtleiter 27 besteht aus einem Glasfaserbündel und ist flexibel ausgebildet.

Wie ebenfalls oben zu Fig. 1 erläutert, tritt die Signalstrahlung von dem beobachteten Bereich der Oberfläche des Hohlraums durch das Fenster 4 in das Endoskopobjektiv 28 ein und wird durch den Strahlteiler 29 in einen Anteil, der auf einen im distalen Endbereich 26 in Längsrichtung des Schafts 22 angeordneten Bildsensor 30 gelangt, und einen weiteren Anteil, der zu einem im Endoskopkopf 25 angeordneten TOF-Bildsensor 31 weitergeleitet wird, aufgeteilt. Zur Weiterleitung des entsprechenden Anteils der Signalstrahlung zum TOF-Bildsensor 31 ist innerhalb des Schafts 22 ein flexibler Bildleiter 32 angeordnet, der aus einem geordneten Bündel von Lichtleitfasern besteht. Auf die distale Endfläche 33 des Bildleiters 32 wird der Anteil der Signalstrahlung über eine Anpassungsoptik 34 abgebildet. Durch die Anpassungsoptik 34 wird eine Anpassung der numerischen Apertur erreicht, um die Lichtleitfasern optimal nutzen zu können. Gemäß einer nicht dargestellten Ausführungsform kann der Bildleiter 32 auf die proximalseitige Austrittsfläche des Strahlteilers 29 aufgekittet sein, wobei der Kleber vorzugsweise einen Brechungsindex gleich dem des Faserkerns oder zwischen dem des Faserkerns und dem des Strahlteilers 29 aufweist. Von der proximalen Endfläche 35 des Bildleiters 32 wird durch eine Abbildungsoptik 36 ein Bild auf der Sensorfläche des TOF-Bildsensors 31 erzeugt. Zur Versorgung des distalen Bildsensors 30 und zur Datenübertragung dient eine elektrische Leitung 37, die ebenfalls innerhalb des Schafts 22 angeordnet ist. Am Endoskopkopf 25 können Lichtleitkabel und elektrische Kabel zur Verbindung des Lichtleiters 27 bzw. der Leitung 37 sowie zur Verbindung des TOF-Bildsensors 31 mit einer nicht dargestellten Steuerungs- und Auswertungseinrichtung angeschlossen werden.

Im distalen Endbereich 26 sind Spulen 38, 38' eines ansonsten nicht dargestellten Lagesensor- bzw. Lageerfassungssystems angeordnet. Die Spulen 38, 38' umgeben den Bildleiter 32 in dessen distalem Endbereich; die Spulen 38, 38' können auf diese Weise innerhalb des Schafts 22 angeordnet werden, ohne dass dieser im Durchmesser wesentlich vergrößert wird. Zumindest im Bereich der Spulen 38, 38' ist der Außenschaft 23 sowie ggf. weitere Umhüllungen oder Verstärkungen nicht-metallisch ausgeführt, um die Funktion des Lagesensorsystems nicht zu beeinträchtigen. Von den Spulen 38, 38' sind nicht dargestellte elektrische Leitungen innerhalb des Schafts 21 zum Endoskopkopf 25 durchgeführt, die ebenfalls keine Vergrößerung des Schaftdurchmessers verursachen. Dadurch, dass die Spulen 38, 38' im distalen Endbereich 26 des Schafts 22 angeordnet sind, stehen die Spulen in einer festen geometrischen Beziehung zum distalen Ende des Schafts, insbesondere zum Endoskopobjektiv 28, zu dem von diesem auf dem distalen Bildsensor 30 erzeugten Bild und zu dem vom Endoskopobjektiv 28 über die Anpassungsoptik 34 auf der distalen Endfläche 33 des Bildleiters 32 erzeugten Bild. Hierdurch ist eine Erfassung der Position und Orientierung des distalen Endbereichs 26 des Schafts 22 möglich und dadurch eine Umrechnung der vom TOF-Bildsensor 31 erfassten 3D-Daten in absolute, auf das Referenzkoordinatensystem des Lageerfassungssystems bezogene 3D-Daten.

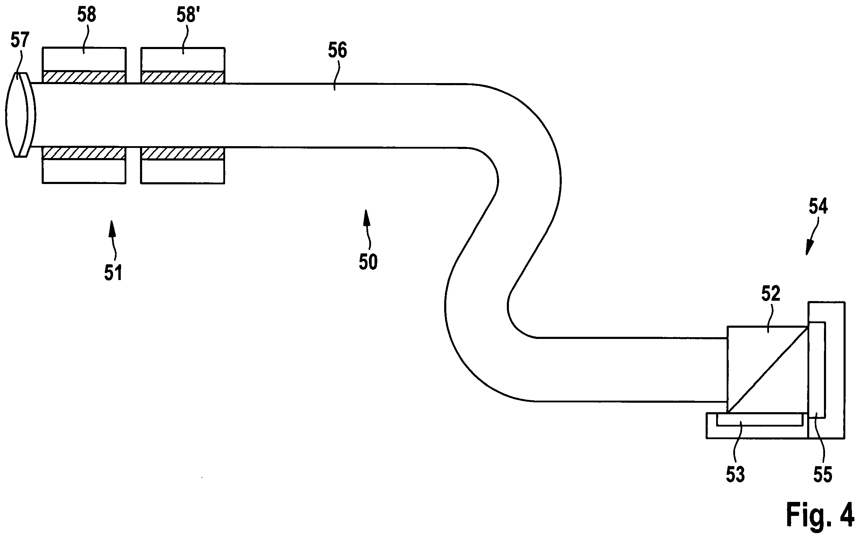

Fig. 4 zeigt in vereinfachter schematischer Darstellung eine weitere Ausführungsform eines flexiblen Endoskops 50 als Teil einer nicht von der Erfindung umfassten Vorrichtung. Die in Fig. 4 gezeigte Ausfuhrungsform unterscheidet sich von der in Fig. 3 dargestellten dadurch, dass ein Strahlteiler 52 und ein Bildsensor 53 nicht im distalen Endbereich 51, sondern im proximalen Endbereich 54 des Endoskops 50 angeordnet sind. Im proximalen Endbereich 54 ist ferner ein TOF-Bildsensor 55 angeordnet. Der Strahlteiler kann beispielsweise einen Anteil der Signalstrahlung zur Erzeugung eines visuellen Bilds eines Bereichs des körperinneren Hohlraums auf den Bildsensor 53 ablenken und den zur Erzeugung der 3D-Daten verwendeten Anteil der Signalstrahlung auf den TOF-Bildsensor 55 durchlassen; die Anordnung des Bildsensors 53 und des TOF-Bildsensors 55 kann auch umgekehrt sein.

Innerhalb eines flexiblen Schafts, der in Fig. 4 nicht dargestellt ist, ist ein flexibler Bildleiter 56 angeordnet, in den durch ein symbolisch dargestelltes Endoskopobjektiv 57 die Signalstrahlung vom beobachteten Bereich eingekoppelt wird. Durch eine im proximalen Endbereich 54 des Endoskops 50 angeordnete, nicht dargestellte Abbildungsoptik sowie ggf. eine Anpassungsoptik werden Bilder des beobachteten Bereichs auf den Sensorflächen des Bildsensors 53 sowie des TOF-Bildsensors 55 erzeugt.

Im distalen Endbereich 51 des Endoskops 50 sind zwei Spulen 58, 58' eines Lageerfassungssystems angeordnet, deren Wicklungen den Bildleiter 56 in raumsparender Anordnung umgeben. Wie zu Fig. 3 erläutert, ist mit den durch das Lageerfassungssystem gelieferten Daten die Erzeugung absoluter 3D-Daten der Oberfläche des beobachteten Hohlraums möglich.

Bei weiteren, nicht dargestellten Ausführungsformen der Vorrichtung, die ansonsten wie in Fig. 3 bzw. Fig. 4 dargestellt ausgebildet sind, ist der TOF-Bildsensor über einen flexiblen Bildleiter mit dem proximalen Endes des Schafts verbindbar. Hierdurch wird die Handhabung des Endoskops wesentlich erleichtert.

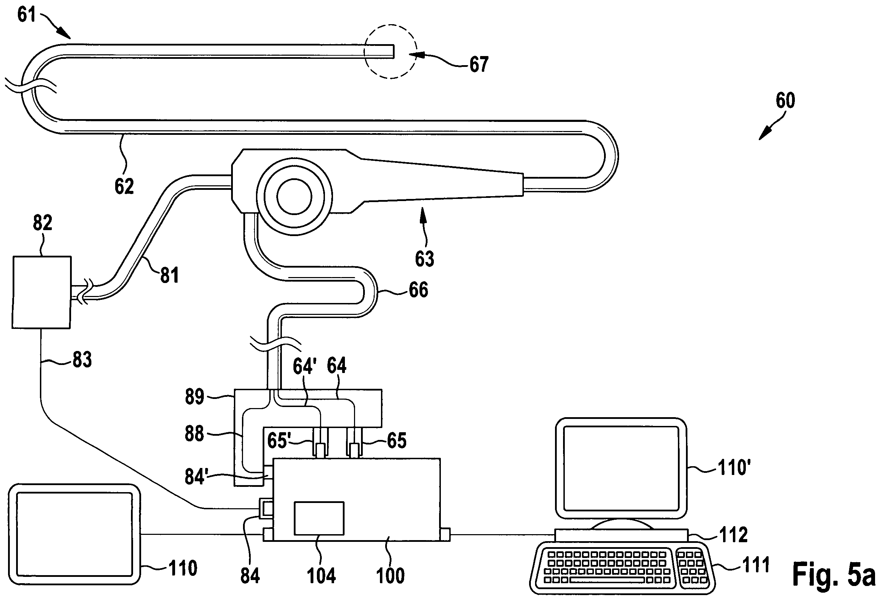

Wie in Fig. 5a dargestellt, umfasst eine erfindungsgemäße Vorrichtung 60 gemäß einem weiteren Ausführungsbeispiel ein Endoskop 61, eine Versorgungseinheit 100 sowie Anzeige- und Eingabeeinrichtungen, etwa Bildschirme 110, 110' und eine Tastatur 111. Das Endoskop umfasst einen flexiblen Schaft 62 und einen Endoskopkopf 63. Die Versorgungseinheit 100 umfasst Lichtquellen zur Erzeugung einer beispielsweise sinusförmig modulierten Messstrahlung und einer Weißlichtbeleuchtung. Zur Weiterleitung beider Strahlungsarten sind jeweils Lichtleitkabel 64, 64' vorgesehen, die über Anschlüsse 65, 65' mit der Versorgungseinheit 100 und über ein Versorgungskabel 66 mit dem Endoskop 61 verbunden werden können. Beide Strahlungsarten können auch über einen einheitlichen Lichtleiter weitergeleitet werden; es können auch jeweils separate Lichtkabel vorgesehen sein. Das Versorgungskabel 66 kann mit dem Endoskopkopf 63 lösbar verbindbar sein, wofür eine entsprechende Kupplung vorgesehen sein kann (nicht dargestellt).

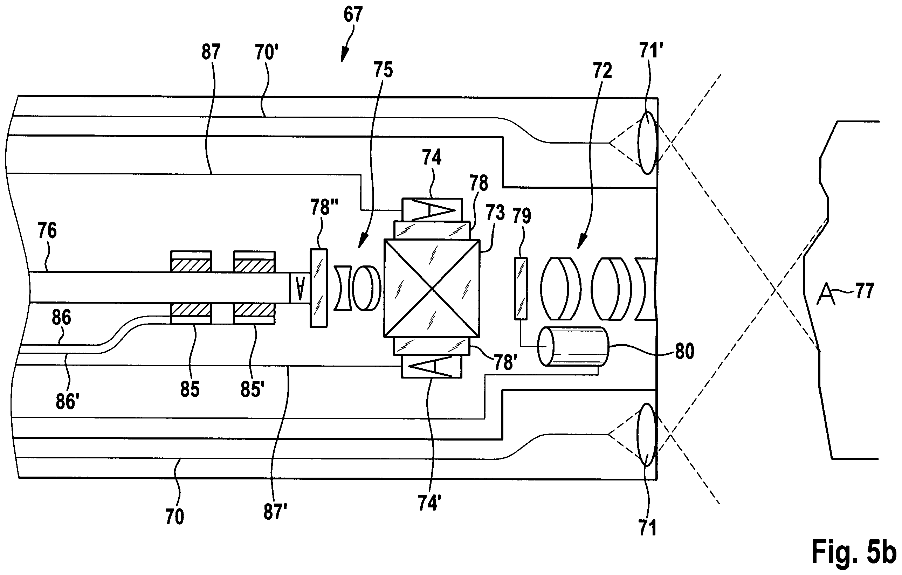

Der distale Endbereich 67 des Endoskops 61, d.h. die Endoskopspitze, ist in Fig. 5b vergrößert dargestellt. Über die Endoskop-Lichtleiter 70, 70' werden die Messstrahlung und das Weißlicht zum distalen Ende des Endoskops geführt. Die dort angeordneten Aufweitungsoptiken 71, 71' dienen der gleichmäßigen Verteilung der Beleuchtungsstrahlung auf einen Teilbereich der Oberfläche eines körperinneren Hohlraums, etwa einen Gewebebereich in dem Hohlraum. Der distale Endbereich 67 enthält ein Endoskopobjektiv 72 zur Erzeugung eines Bilds des Gewebebereichs, einen als Teilerwürfel 73 ausgebildeten Strahlteiler zur Aufteilung des Bilds, einen oder mehrere Bildsensoren 74, 74' sowie ggf. eine oder mehrere Anpassungsoptiken 75, hier exemplarisch bei dem Bildleiter 76 dargestellt. Die Anpassungsoptik 75 ist so ausgebildet, dass auf die distale Endfläche des Bildleiters 76 trotz unterschiedlicher Größe das gleiche Bildfeld abgebildet wird wie auf die Bildsensoren 74, 74'. Dies ist in Fig. 5b symbolisch durch die abgebildete Struktur 77 dargestellt, die durch die als Verkleinerungsoptik ausgebildete Anpassungsoptik auf die Endfläche des geordneten Faserbündels des Bildleiters 76 kleiner als auf die Bildsensoren 74, 74' abgebildet wird. Ferner können Aufbereitungsoptiken vorhanden sein, die beispielsweise als Filter 78, 78', 78", etwa als Farbfilter, elektronisch abstimmbare Filter, Prismen, Verlängerungsplatten oder Raumfrequenzfilter (Anti-Aliasing Filter) ausgebildet sein können. Ferner kann ein ein- und ausschwenkbarer Filter 79 mit einem Aktuator 80 vorgesehen sein.

Das vom Endoskopobjektiv 72 auf der distalen Endfläche des Bildleiters 76 erzeugte Bild wird von dem Bildleiter 76 zum Endoskopkopf 63 geleitet und über den dort angekoppelten Bildleiter 81 zu einer TOF-Kameraeinheit 82, die einen TOF-Bildsensor sowie eine Abbildungsoptik zur Erzeugung eines Bilds auf der Sensorfläche des TOF-Bildsensors enthält (s. Fig. 5a). Der Bildleiter 76 kann mit dem Bildleiter 81 auch einstückig ausgebildet sein, wodurch ein Lichtverlust an der Koppelstelle vermieden werden kann. Wird statt des dargestellten flexiblen Endoskops 61 ein starres Endoskop verwendet, so kann das erzeugte Bild auch über Relaislinsensysteme zum Endoskopkopf übertragen werden. Die TOF-Kameraeinheit 82 ist über ein elektrisches Kabel 83 und den Anschluss 84 an die Versorgungseinheit 100 anschließbar.

Wie in Fig. 5b symbolisch angedeutet, sind am Bildleiter 76 in dessen distalem Endbereich in Längsrichtung des distalen Endbereichs 67 versetzt zwei Spulen 85, 85' angeordnet, deren Wicklungen den Bildleiter 76 umgeben. Die Spulen 85, 85' stellen die Sensormittel eines nicht dargestellten elektromagnetischen Lageerfassungssystems dar. Das Lageerfassungssystem erzeugt ein derart ausgebildetes äußeres Magnetfeld, dass aus den von den Spulen 85, 85' gelieferten Strom- bzw. Spannungssignalen die Position der Spulen 85, 85' innerhalb des Magnetfelds, d.h. relativ zu einem äußeren, von der Lage des Endoskops 61 unabhängigen Koordinatensystem, ermittelbar ist. Aus der Differenz zwischen den Signalen der beiden Spulen 85, 85' kann die Orientierung des distalen Endbereichs 67 des Schafts 62 und somit die Blickrichtung ermittelt werden. Die Signale der Spulen 85, 85' werden über elektrische Leitungen 86, 86' zum Endoskopkopf 63 übertragen. Zur Versorgung und Signalübertragung der Bildsensoren 74, 74' dienen die weiteren Leitungen 87, 87', wobei im distalen Endbereich 67 oder im Endoskopkopf 63 elektronische Komponenten zur Steuerung der Bildsensoren 74, 74' angeordnet sein können. Über die in Fig. 5a symbolisch dargestellte Leitung 88 und den Anschluss 84' sind die Spulen 85, 85' und die Bildsensoren 74, 74' sowie ggf. weitere elektrische Einrichtungen des Endoskops 61 mit der Versorgungseinheit 100 verbunden. Die Lichtleiter 64, 64' und die Leitung 88 können in einer Anschlussbox 89 zusammengefasst sein.

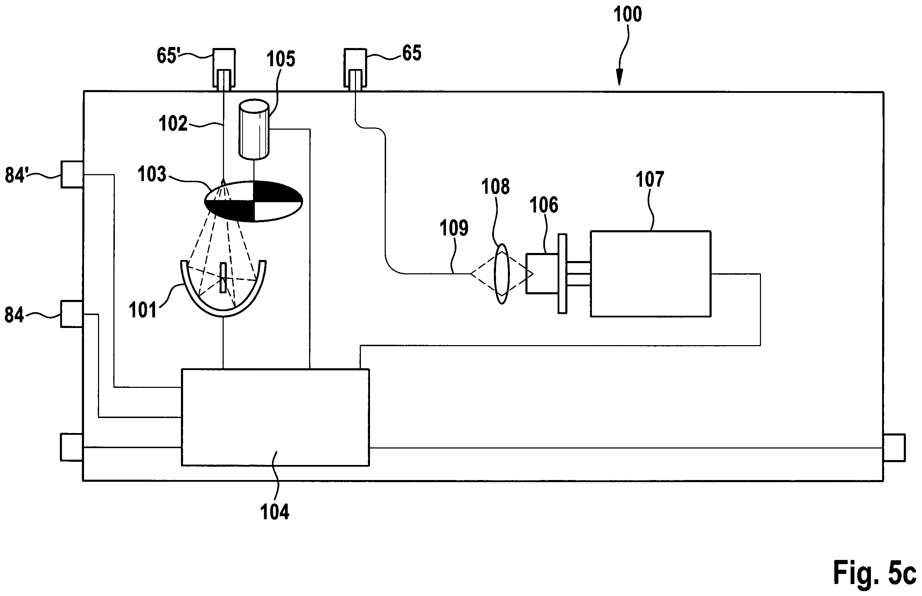

In Fig. 5c ist die Versorgungseinheit 100 schematisch dargestellt. Zur Erzeugung einer Weißlichtbeleuchtung enthält die Versorgungseinheit eine Metall-Halid-Bogen-Entladungslampe 101, die einen Reflektor umfassen kann, sowie weitere Elemente zur Kollimation bzw. Einkopplung in einen Lichtleiter 102. Alternativ können auch LED-, Xenon- oder Halogenlampen als Weißlichtquelle verwendet werden, ebenso wie RGB- oder Superkontinuum-Laserquellen. Ferner kann ein Wärmeschutzfilter vorgesehen sein (nicht dargestellt). Um zu verhindern, dass das Weißlicht die Tiefenmessung beeinträchtigt, ist ein Chopperrad 103 vorgesehen, das den Lichtfluss unterbricht, sobald eine Abstandsdatenerfassung stattfindet. Dies kann manuell eingegeben werden, um abwechselnd im Weißlicht und im Messlicht zu beobachten oder auch ein Fluoreszenzbild aufzunehmen. Es kann aber auch automatisch, insbesondere im Videotakt oder einem Bruchteil davon, zwischen Weißlicht bzw. Fluoreszenzbeobachtung und 3D-Messung umgeschaltet werden. Das Steuergerät 104 steuert den Antrieb 105 des Chopperrads entsprechend den jeweiligen Anforderungen, beispielsweise synchron mit dem Auslesen des jeweiligen Bildsensors. Anstelle eines Chopperrads kann auch ein Schwingspiegel oder ein elektronisch gesteuertes Filter verwendet werden. Bei Verwendung von Festkörperlichtquellen, beispielsweise LED- oder Laserlichtquellen, können diese direkt im entsprechenden Takt angesteuert werden. Der Lichtleiter 102 leitet das Licht über einen Anschluss 65' in den Lichtleiter 64' ein.

Zur Erzeugung der Messstrahlung ist eine Laserdiode 106 vorgesehen, die über eine Treiberelektronik 107 angesteuert wird und deren Licht über eine Kollimationsoptik 108 in einen Lichtleiter 109 eingekoppelt wird. Stattdessen kann auch eine fasergekoppelte Leuchtdiode oder eine Superlumineszenzdiode eingesetzt werden. Ferner können Mittel zur Verringerung der Kohärenz der Messstrahlung vorgesehen sein. Das erzeugte Licht wird über einen Anschluss 65 in den Lichtleiter 64 zur Weiterleitung in das Endoskop 61 eingeleitet (s. Fig. 5a). Die Laserdiode wird durch das Steuergerät 104 synchron zum Auslesen des phasenempfindlichen Bildsensors moduliert.

Das Weißlicht und die Messstrahlung können auch in einen gemeinsamen Lichtleiter eingekoppelt werden. Ferner kann Fluoreszenzanregungslicht, das von der Lichtquelle 101 erzeugt werden kann, beispielsweise über einen im Endoskopkopf angeordneten Strahlteiler oder in einer die TOF-Kameraeinheit 82 enthaltenden, integrierten Versorgungseinheit in den Bildleiter 81 eingekoppelt und durch diesen zum distalen Endbereich 67 des Endoskops 61 geleitet werden (nicht dargestellt).

Das Steuergerät 104 dient auch zur Ansteuerung der TOF-Kameraeinheit 82 und zur Auswertung der von dieser bzw. vom TOF-Bildsensor gelieferten Signale. Der TOF-Bildsensor registriert pixelweise die Intensität der auftreffenden Signalstrahlung und die Phasenverschiebung, d. h., die Zeitverzögerung zwischen der emittierten Messstrahlung und der aufgenommenen Signalstrahlung. Durch eine Auswertung, wie beispielsweise in EP 1 746 410 A1 angegeben, kann pixelweise die Phasenverschiebung und damit die Zeitverzögerung, die der Lichtlaufzeit entspricht, ermittelt werden. Aus der Zeitverzögerung können auf den Endbereich 67 des Endoskops 61 bezogene, d.h. relative 3D-Daten rekonstruiert werden.

Ferner sind auch die Bildsensoren 74, 74' mit dem Steuergerät 104 verbunden, das die Bildsignale ausliest bzw. für eine Anzeige verarbeitet, das Chopperrad 103 und die Laserdiode 106 entsprechend synchron ansteuert und die Bilddaten zur weiteren Verarbeitung, Anzeige und Speicherung weiter an einen Computer 112 weiterleitet. Weiterhin ist das Steuergerät 104 zur Verarbeitung der Signale der Spulen 85, 85' bzw. zur Kommunikation mit dem Lageerfassungssystem ausgebildet. Die hierdurch gewonnenen Lagedaten ermöglichen die Bestimmung der Position und Orientierung des distalen Endbereichs 67 des Endoskops 61 relativ zu einem durch das Lageerfassungssystem gegebenen extrakorporalen Koordinatensystem. Das Steuergerät 104 ermittelt hieraus sowie aus den relativen 3D-Daten, die aus den Signalen des TOF-Bildsensors ermittelt werden, absolute, d.h. auf das extrakorporale Koordinatensystem bezogene 3D-Daten des beobachteten Teilbereichs der Oberfläche des körperinneren Hohlraums. Diese Daten werden an den Computer 112 übermittelt.

Durch Verknüpfung von 3D-Daten, die bei unterschiedlichen Positionen und Orientierungen des Endoskops 61 bzw. dessen distalen Endbereichs 67 aufgenommen worden sind, ist eine praktisch vollständige Erfassung der inneren Oberfläche des Hohlraums möglich, sowie die Erstellung eines virtuellen 3D-Modells des körperinneren Hohlraums. Die erzeugten absoluten 3D-Daten können auch zur Längen-, Flächen- oder Volumenmessung ausgewertet werden. Ferner ist im Steuergerät 104 oder im Computer 112 eine Möglichkeit zur Verknüpfung bzw. synoptischen Darstellung der vom TOF-Bildsensor sowie von den Bildsensoren 74, 74' gelieferten unterschiedlichen Bilddaten vorgesehen, sowie ggf. die Erzeugung eines synthetischen Stereobilds. Die Bilddaten können auf den Bildschirmen 110, 110' dargestellt werden. Ferner ist eine Eingabeeinrichtung zur Eingabe von Anweisungen eines Benutzers vorhanden, beispielsweise eine Tastatur 111, ein Touch Screen oder auch eine Spracherkennungseinrichtung.

Eine TOF-Kameraeinheit 82 und/oder eine Versorgungseinheit 100 wie vorstehend beschrieben können auch im Zusammenhang mit Endoskopen, die gemäß Fig. 1 bis Fig. 4 ausgebildet sind, verwendet werden. Dabei, wie auch in der Ausführungsform gemäß Fig. 5a bis 5c, kann die TOF-Kameraeinheit 82 auch in die Versorgungseinheit 100 integriert sein. In diesem Fall verläuft der Bildleiter 81 vorzugsweise in dem Versorgungskabel 66 und kann über ein Stecksystem in die Versorgungseinheit 100 eingeführt werden (nicht dargestellt). Hierdurch entsteht eine besonders kompakte und leicht bedienbare Vorrichtung. Auch die Anordnung aus Strahlteiler 52, Bildsensor 53 und TOF-Bildsensor 55 in der Ausführungsform nach Fig. 4 kann in die Versorgungseinheit integriert sein und über einen vorzugsweise durch ein Versorgungskabel geführten Bildleiter mit dem Endoskop 50 verbunden sein.

Für die Anwendung eines nicht von der Erfindung umfassten Verfahrens zur endoskopischen 3D-Datenerfassung wird das Endoskop 1, 21, 50, 61 in die Körperhöhle, worin die Untersuchung bzw. der Eingriff erfolgen soll, eingeführt. Der TOF-Bildsensor wird, sofern er nicht eine Einheit mit dem Endoskop darstellt oder bereits mit diesem verbunden ist, an das Endoskop 1, 21, 50, 61 angeschlossen, beispielsweise über einen Bildleiter 81 und eine entsprechende Kupplung. Sofern die Lichterzeugungsmittel nicht Teil des Endoskops sind, werden eine Lichtquelle bzw. die Versorgungseinheit 100, die Lichterzeugungsmittel zur Erzeugung einer Messstrahlung enthält, mit dem Endoskop 1, 21, 50, 61 über ein Lichtkabel bzw. das Versorgungskabel 66 verbunden. Eine Befestigung des Endoskops 1, 21, 50, 61 an einem Halter, der eine Bewegung des Endoskops relativ zum untersuchten Patienten bzw. relativ zum Hohlraum während der Untersuchung verhindert, ist aufgrund der Erfassung absoluter 3D-Daten in der Regel nicht notwendig.

Zur Durchführung des Verfahrens zur 3D-Datenerfassung wird in der Versorgungseinheit 100 das Beleuchtungslicht erzeugt, insbesondere die Messstrahlung und Weißlicht. Die Messstrahlung ist mit einer Frequenz von beispielsweise ca. 10 - 100 MHz sinusförmig intensitätsmoduliert. Das Weißlicht kann beispielsweise das ganze sichtbare Spektrum oder einen Teil davon umfassen, kann aber auch aus einem oder mehreren schmalbandigen Anteilen bestehen. Vorteilhafterweise wird das Weißlicht im Videotakt geschaltet.

Weißlicht und Messstrahlung werden über Lichtleiter 7, 26, 70, 70' auf das zu beobachtende Gebiet geleitet. Über die Beobachtungsoptik 9, 28, 57, 72 und den Bildweiterleiter 12 bzw. den Bildleiter 32, 56, 76, 81 wird auf dem TOF-Bildsensor 15, 31, 75 ein Bild erzeugt. Durch mit der Modulation der Messstrahlung synchronisiertes Auslesen des Bildsensors werden pixelweise phasenabhängige Daten gewonnen, die von dem Steuergerät 104 zu Intensitäts- und Phaseninformationen verarbeitet werden. Das Steuergerät 104 erzeugt dadurch Tiefeninformationen, die die Zeitverzögerung der Signalstrahlung gegenüber der Messstrahlung darstellen, und daraus wiederum 3D-Daten. Das Signal des TOF-Bildsensors kann auch zur Erzeugung eines Fluoreszenzbilds verwendet werden; ferner können durch die weiteren Bildsensoren 11, 30, 74, 74' weitere Bilder erzeugt werden. Weiterhin werden die Signale der Lagesensormittel, insbesondere der Spulen 38, 38', 58, 58', 85, 85' vom Steuergerät aufgenommen und zur Erzeugung absoluter 3D-Daten verwendet. Die 3D-Daten sowie ggf. die weiteren Bilder können für den Benutzer auf geeigneten Anzeigeeinrichtungen 110, 110' dargestellt werden und für weitere Bildverarbeitungsschritte oder für die Speicherung zur Verfügung stehen. So können etwa ein RGB- oder ein Fluoreszenzbild abwechselnd oder in Überlagerung mit den 3D-Daten dargestellt werden.

Sofern keine 3D-Datenerfassung gewünscht ist, kann auch ein Betrieb ohne TOF-Sensor vorgesehen sein, wobei beispielsweise der Bildleiter 81 nicht mit dem Endoskop 61 verbunden ist. Hierbei kann wie bei einem Standard-Videoendoskop beispielsweise lediglich ein visuelles Bild eines Teilbereichs des körperinneren Hohlraums erzeugt werden.

### Bezugszeichenliste

- 1: Endoskop
- 2: Schaft
- 3: Rohr
- 4, 4': Fenster
- 5: Proximaler Endbereich
- 6: Lichtanschlussstutzen
- 7: Lichtleiter
- 8: Distaler Endbereich
- 9: Endoskopobjektiv
- 10: Strahlteiler
- 11: Bildsensor
- 12: Bildweiterleiter
- 13, 13', 13": Stablinse
- 14: Abbildungsoptik
- 15: TOF-Bildsensor
- 16: Leitung
- 21: Endoskop
- 22: Schaft
- 23: Außenschaft
- 24, 24': Fenster
- 25: Endoskopkopf
- 26: Distaler Endbereich
- 27: Lichtleiter
- 28: Endoskopobjektiv
- 29: Strahlteiler
- 30: Bildsensor
- 31: TOF-Bildsensor
- 32: Bildleiter
- 33: Distale Endfläche
- 34: Anpassungsoptik
- 35: Proximale Endfläche
- 36: Abbildungsoptik
- 37: Leitung
- 38, 38': Spule
- 40: Endoskopobjektiv
- 41: Strahlteiler
- 42, 42': Stablinse
- 43: Relaislinsensystem
- 44: Verlängerung
- 45: Planparallele Platte
- 50: Endoskop
- 51: Distaler Endbereich
- 52: Strahlteiler
- 53: Bildsensor
- 54: Proximaler Endbereich
- 55: TOF-Bildsensor
- 56: Bildleiter
- 57: Endoskopobjektiv
- 58, 58': Spule
- 60: Vorrichtung
- 61: Endoskop
- 62: Schaft
- 63: Endoskopkopf
- 64, 64': Lichtleiter
- 65, 65': Anschluss
- 66: Versorgungskabel
- 67: Distaler Endbereich
- 70, 70': Endoskop-Lichtleiter
- 71, 71': Aufweitungsoptik
- 72: Endoskopobjektiv
- 73: Teilerwürfel
- 74, 74': Bildsensor
- 75: Anpassungsoptik
- 76: Bildleiter
- 77: Struktur
- 78, 78', 78": Filter
- 79: Filter
- 80: Aktuator
- 81: Bildleiter
- 82: TOF-Kameraeinheit
- 83: Kabel
- 84, 84': Anschluss
- 85, 85': Spule
- 86, 86': Leitung
- 87, 87': Leitung
- 88: Leitung
- 89: Anschlussbox
- 100: Versorgungseinheit
- 101: Lampe
- 102: Lichtleiter
- 103: Chopperrad
- 104: Steuergerät
- 105: Antrieb
- 106: Laserdiode
- 107: Treiberelektronik
- 108: Kollimatoroptik
- 109: Lichtleiter
- 110, 110': Bildschirm
- 111: Tastatur
- 112: Computer

## Patentansprüche

1. Vorrichtung zur endoskopischen 3D-Datenerfassung, umfassend Lichterzeugungsmittel zur Erzeugung mindestens einer modulierten Messstrahlung, Lichtweiterleitungsmittel eingerichtet zur Weiterleitung der Messstrahlung auf zumindest einen Teilbereich einer Oberfläche eines körperinneren Hohlraums, die zumindest teilweise in einem endoskopisch einsetzbaren langerstreckten Schaft (3, 23, 62) angeordnet sind, eine in einem distalen Endbereich (8, 26, 51, 67) des Schafts angeordnete Beobachtungsoptik eingerichtet zur Aufnahme einer Signalstrahlung von zumindest dem Teilbereich der Oberfläche des Hohlraums, einen Time-of-Flight- (TOF-) Bildsensor (15, 31, 55), einen zumindest teilweise innerhalb des Schafts (3, 23, 62) angeordneten Bildweiterleiter eingerichtet zum Weiterleiten der Signalstrahlung vom distalen zu einem proximalen Endbereich des Schafts zur Aufnahme durch den TOF-Bildsensor (15, 31, 55) und Steuerungs- und Auswertungsmittel eingerichtet zur Ansteuerung der Lichterzeugungsmittel zur Erzeugung der Messstrahlung, zur Ansteuerung des TOF-Bildsensors (15, 31, 55) und zur Auswertung von von dem TOF-Bildsensor gelieferten räumlich aufgelösten Abstands- bzw. Tiefendaten von Messpunkten auf der Oberfläche des Hohlraums zur Erzeugung von 3D-Daten, wobei die Vorrichtung Lagesensormittel eingerichtet zur Erfassung von Informationen zu einer Position und einer Orientierung des Schafts (3, 23, 62) umfasst und wobei die Steuerungs- und Auswertungsmittel dazu ausgebildet sind, aufgrund der mit Hilfe der Lagesensormittel erfassten Informationen aus den vom TOF-Bildsensor (15, 31, 55) gelieferten Daten 3D-Daten zu berechnen, die die Oberfläche des Hohlraums in Bezug zu einem absoluten, von der Lage des Schafts unabhängigen, extrakorporalen Referenzkoordinatensystem dreidimensional darstellen, **dadurch gekennzeichnet, dass** die Lagesensormittel als elektromagnetischer Lagesensor ausgebildet sind, der mindestens zwei Spulen (38, 38', 58, 58', 85, 85') umfasst, wobei der TOF-Bildsensor (15, 31, 55), der zur Ermittlung der Abstandsdaten ein Bild im infraroten Spektralbereich aufnimmt, über einen flexiblen Bildleiter (81) mit dem proximalen Endbereich des Bildweiterleiters lösbar verbindbar ist und wobei im distalen Endbereich (8, 26, 67) des Schafts (3, 23, 62) ein distaler Strahlteiler (10, 29, 73) und ein weiterer Bildsensor (11, 30, 74, 74') eingerichtet zur Erfassung eines weiteren Bildes des Teilbereichs der Oberfläche des Hohlraums, das ein visuelles Bild ist, angeordnet sind.

2. Vorrichtung nach Anspruch 1, **dadurch gekennzeichnet, dass** der Schaft (3) starr ausgebildet ist.

3. Vorrichtung nach Anspruch 1, **dadurch gekennzeichnet, dass** der Schaft (23, 62) flexibel ausgebildet ist, dass der Bildweiterleiter ein flexibler Bildleiter (32, 56, 76) ist und dass die Lagesensormittel im distalen Endbereich des Schafts angeordnet sind.

4. Vorrichtung nach einem der vorhergehenden Ansprüche, **dadurch gekennzeichnet, dass** die Lagesensormittel einen Positionssensor, insbesondere einen Positions- und Orientierungssensor umfassen.

5. Vorrichtung nach einem der vorhergehenden Ansprüche, **dadurch gekennzeichnet, dass** die Spulen (38, 38', 58, 58', 85, 85') den Bildweiterleiter umgeben.

6. Vorrichtung nach einem der vorhergehenden Ansprüche, **dadurch gekennzeichnet, dass** die Vorrichtung eine Bildverarbeitungseinrichtung umfasst, die ausgebildet ist, um den Bildpunkten des von dem weiteren Bildsensor (11, 30, 74, 74') aufgenommenen Bilds jeweils eine Tiefeninformation zuzuordnen, die aus den von dem TOF-Bildsensor (15, 31, 55) gelieferten Daten gewonnen wird.

7. Vorrichtung nach einem der vorhergehenden Ansprüche, **dadurch gekennzeichnet, dass** mit dem proximalen Endbereich des Bildweiterleiters ein proximaler Strahlteiler (52) verbunden oder verbindbar ist, an den ein Spektralanalysator ankoppelbar ist.

8. Vorrichtung nach einem der vorhergehenden Ansprüche, **dadurch gekennzeichnet, dass** der Bildweiterleiter zum Weiterleiten einer Fluoreszenzanregungsstrahlung vom proximalen zum distalen Endbereich (8, 26, 51, 67) des Schafts (3, 23, 62) ausgebildet ist.

## Claims

1. Apparatus for endoscopic 3D data capture, comprising light-generation means for generating at least one modulated measurement radiation, light-transmission means configured to carry the measurement radiation to at least one portion of a surface of a body-internal cavity, which light-transmission means are at least partially disposed in an elongate shaft (3, 23, 62) that can be used in endoscopic fashion, an observation optical unit disposed in a distal end region (8, 26, 51, 67) of the shaft, which observation optical unit is configured to record signal radiation from at least the portion of the surface of the cavity, a time-of-flight (TOF) image sensor (15, 31, 55), an image carrier disposed at least partially within the shaft (3, 23, 62), which image carrier is configured to carry the signal radiation from the distal to a proximal end region of the shaft for reception by the TOF image sensor (15, 31, 55), and control and evaluation means configured to actuate the light-generation means to generate the measurement radiation, to actuate the TOF image sensor (15, 31, 55) and to evaluate spatially resolved distance or depth data of measurement points on the surface of the cavity, supplied by the TOF image sensor, to generate 3D data, wherein the apparatus comprises position and orientation sensor means, which are configured to capture information items about a position and an orientation of the shaft (3, 23, 62) and wherein the control and evaluation means are embodied to calculate 3D data on the basis of the information items, captured with the aid of the position and orientation sensor means, from the data supplied by the TOF image sensor (15, 31, 55), said 3D data representing the surface of the cavity in three dimensions in relation to an absolute extracorporeal reference coordinate system that is independent of the position of the shaft, **characterized in that** the position and orientation sensor means are embodied as an electromagnetic position and orientation sensor comprising at least two coils (38, 38', 58, 58', 85, 85'), wherein the TOF image sensor (15, 31, 55), which records an image in the infrared spectral range for the purposes of ascertaining the distance data, is detachably connectable via a flexible image conductor (81) to the proximal end region of the image carrier and wherein, disposed in the distal end region (8, 26, 67) of the shaft (3, 23, 62), there is a distal beam splitter (10, 29, 73) and a further image sensor (11, 30, 74, 74') configured to capture a further image of the portion of the surface of the cavity, said image being a visual image.

2. Apparatus according to Claim 1, **characterized in that** the shaft (3) has a rigid embodiment.

3. Apparatus according to Claim 1, **characterized in that** the shaft (23, 62) has a flexible embodiment, **in that** the image carrier is a flexible image conductor (32, 56, 76) and **in that** the position and orientation sensor means are disposed in the distal end region of the shaft.

4. Apparatus according to any one of the preceding claims, **characterized in that** the position and orientation sensor means comprise a position sensor, more particularly a position and orientation sensor.

5. Apparatus according to any one of the preceding claims, **characterized in that** the coils (38, 38', 58, 58', 85, 85') surround the image carrier.

6. Apparatus according to any one of the preceding claims, **characterized in that** the apparatus comprises an image processing device configured to assign to the pixels of the image recorded by the further image sensor (11, 30, 74, 74') a respective depth information item, said depth information item being obtained from the data supplied by the TOF image sensor (15, 31, 55).

7. Apparatus according to any one of the preceding claims, **characterized in that** a proximal beam splitter (52), to which a spectral analyser is coupleable, is connected or connectable to the proximal end region of the image carrier.

8. Apparatus according to any one of the preceding claims, **characterized in that** the image carrier is embodied to carry fluorescence excitation radiation from the proximal to the distal end region (8, 26, 51, 67) of the shaft (3, 23, 62).

## Revendications

1. Dispositif d'acquisition de données 3D endoscopiques, comprenant des moyens de génération de lumière destinés à générer au moins un rayonnement de mesure modulé, des moyens de retransmission de lumière conçus pour retransmettre le rayonnement de mesure sur au moins une zone partielle d'un espace vide intracorporel, lesquels sont au moins partiellement disposés dans une tige (3, 23, 62) étendue en longueur utilisable pour une endoscopie, une optique d'observation disposée dans une zone d'extrémité distale (8, 26, 51, 67) de la tige et conçue pour enregistrer un rayonnement de signal d'au moins une zone partielle de la surface de l'espace vide, un capteur d'images (15, 31, 55) à temps de vol (TOF), un retransmetteur d'images disposé au moins partiellement à l'intérieur de la tige (3, 23, 62) et destiné à retransmettre le rayonnement de signal de la zone d'extrémité distale à une zone d'extrémité proximale de la tige en vue de l'enregistrement par le capteur d'images TOF (15, 31, 55) et des moyens de commande et d'interprétation conçus pour commander les moyens de génération de lumière en vue de générer le rayonnement de mesure, pour commander le capteur d'images TOF (15, 31, 55) et pour interpréter des données d'écart ou de profondeur à résolution spatiale délivrées par le capteur d'images TOF sur la surface de l'espace vide en vue de générer des données 3D, le dispositif comportant des moyens de détection de position conçus pour détecter des informations relatives à une position et une orientation de la tige (3, 23, 62) et les moyens de commande et d'interprétation étant configurés pour, à l'aide des informations détectées par les moyens de détection de position, calculer des données 3D à partir des données délivrées par le capteur d'images TOF (15, 31, 55), lesquelles représentent en trois dimensions la surface de l'espace vide en référence à un système de coordonnées de référence extracorporel absolu indépendant de la position de la tige, **caractérisé en ce que** les moyens de détection de position sont réalisés sous la forme d'un capteur de position électromagnétique, lequel comporte au moins deux bobines (38, 38', 58, 58', 85, 85'), le capteur d'images TOF (15, 31, 55), qui enregistre une image dans la plage spectrale infrarouge en vue de déterminer les données d'écart, pouvant être relié de manière amovible à la zone d'extrémité proximale du retransmetteur d'images par le biais d'un conducteur optique (81) flexible et un diviseur de faisceau (10, 29, 73) distal et un capteur d'images supplémentaire (11, 30, 74, 74'), conçu pour acquérir une image supplémentaire de la zone partielle de la surface de l'espace vide, laquelle est une image visuelle, étant disposés dans la zone d'extrémité distale (8, 26, 67) de la tige (3, 23, 62).

2. Dispositif selon la revendication 1, **caractérisé en ce que** la tige (3) est de configuration rigide.

3. Dispositif selon la revendication 1, **caractérisé en ce que** la tige (23, 62) est de configuration flexible, **en ce que** le retransmetteur d'images est un conducteur optique flexible (32, 56, 76) et **en ce que** les moyens de détection de position sont disposés dans la zone d'extrémité distale de la tige.

4. Dispositif selon l'une des revendications précédentes, **caractérisé en ce que** les moyens de détection de position comportent un détecteur de position, notamment un détecteur de position et d'orientation.

5. Dispositif selon l'une des revendications précédentes, **caractérisé en ce que** les bobines (38, 38', 58, 58', 85, 85') entourent le retransmetteur d'images.

6. Dispositif selon l'une des revendications précédentes, **caractérisé en ce que** le dispositif comporte un appareil de traitement d'images qui est configuré pour associer respectivement une information de profondeur aux pixels de l'image enregistrée par le capteur d'images supplémentaire (11, 30, 74, 74'), laquelle est obtenue à partir des données délivrées par le capteur d'images TOF (15, 31, 55).

7. Dispositif selon l'une des revendications précédentes, **caractérisé en ce qu'**un diviseur de faisceau proximal (52), auquel peut être couplé un analyseur spectral, est relié ou peut être relié à la zone d'extrémité proximale du retransmetteur d'images.

8. Dispositif selon l'une des revendications précédentes, **caractérisé en ce que** le retransmetteur d'images est configuré pour retransmettre un rayonnement d'excitation en fluorescence de la zone d'extrémité proximale à la zone d'extrémité distale (8, 26, 51, 67) de la tige (3, 23, 62).
